(19) Europäisches Patentamt
European Patent Office
Office européen des brevets

(11) Numéro de publication: **0 315 518 B1**

(12) **FASCICULE DE BREVET EUROPEEN**

(45) Date de publication de fascicule du brevet: **16.02.94**

(51) Int. Cl.5: **C07D 501/24**, A61K 31/545

(21) Numéro de dépôt: **88402740.0**

(22) Date de dépôt: **02.11.88**

Le dossier contient des informations techniques présentées postérieurement au dépôt de la demande et ne figurant pas dans le présent fascicule.

# (54) Céphalosporines comportant en position 3 un radical vinyle substitué, leur procédé de préparation, leur application comme médicaments, les compositions les renfermant et les intermédiaires obtenus.

(30) Priorité: **03.11.87 FR 8715210**

(43) Date de publication de la demande: **10.05.89 Bulletin 89/19**

(45) Mention de la délivrance du brevet: **16.02.94 Bulletin 94/07**

(84) Etats contractants désignés: **AT BE CH DE ES FR GB GR IT LI LU NL SE**

(56) Documents cités:
**GB-A- 2 134 522**
**GB-A- 2 157 293**

**PATENT ABSTRACTS OF JAPAN, vol. 9, no. 28 (C-264)[1751], 6 février 1985, page 14 C 264**

(73) Titulaire: **ROUSSEL-UCLAF**
**35, Boulevard des Invalides**
**F-75007 Paris(FR)**

(72) Inventeur: **Aszodi, Joszef**
**60, Ouai Pompadour**
**F-94600 Choisy-Le-Roi(FR)**
Inventeur: **Bonnet, Alain**
**19, Allée Lucien Michard**
**F-93190 Livry-Gargan(FR)**
Inventeur: **Chantot, Jean-François**
**2, Allée Eole**
**F-77410 Gressy-En-France(FR)**

(74) Mandataire: **Bourgouin, André et al**
**Département des Brevets**
**ROUSSEL UCLAF**
**111, route de Noisy**
**B.P. no 9**
**F-93230 Romainville (FR)**

EP 0 315 518 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles céphalosporines comportant en position 3 un radical vinyle substitué, leur procédé de préparation, leur application comme médicaments, les compositions les renfermant et les nouveaux intermédiaires obtenus.

Dans le brevet britannique GB 2134522 sont décrits des produits pouvant comporter notamment un substituant thiazolo-pyridinium en position 3.

L'invention a pour objet les produits de formule générale (I) :

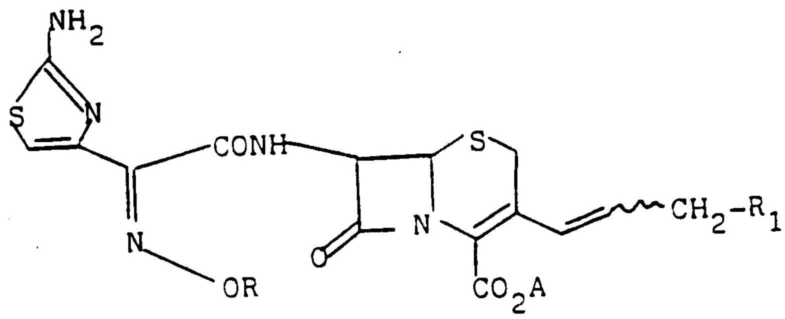

(I)

isomère syn

dans laquelle R représente un atome d'hydrogène ou un radical alkyle, linéaire ou ramifié, alkényle, alkynyle ou cycloalkyle ayant au plus 6 atomes de carbone, chacun de ces radicaux étant éventuellement substitué par un ou plusieurs des radicaux suivants :

carboxy éventuellement salifié ou estérifié, méthoxycarbonyle, éthoxycarbonyle, carbamoyle, diméthylcarbamoyle ; amino ; diméthylamino ; méthylamino ; halogène ; méthoxy, éthoxy, propyloxy ; méthylthio, éthylthio ; phényle ; tétrazolyle ; phénylthio ; tétrazolylthio, thiadiazolylthio éventuellement substitué par méthyle ; $R_1$ représente un radical choisi parmi :

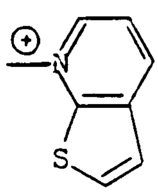 ; 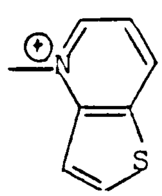 , 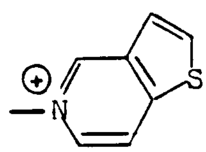 et 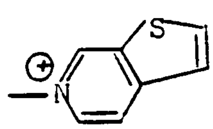

A représente un atome d'hydrogène, un équivalent de métal alcalin, alcalino-terreux, de magnésium, d'ammonium ou d'une base organique aminée ou A représente le reste d'un groupement ester facilement clivable ou $CO_2$ A représente $CO_2^{-}$ le trait ondulé signifie que le groupement $CH_2R_1$ peut se trouver dans la position E ou Z, ainsi que les sels des produits de formule (I) avec les acides minéraux ou organiques.

L'expression alkyle, linéaire ou ramifié, ayant au plus 6 atomes de carbone inclut les radicaux méthyle, éthyle, propyle, isopropyle, butyle, isobutyle, sec-butyle, tert-butyle, pentyle, isopentyle, sec-pentyle, tert-pentyle, néopentyle, hexyle, isohexyle, sec-hexyle, tert-hexyle.

L'expression alkényle ayant au plus 6 atomes de carbone inclut les radicaux vinyle, allyle, 1-propényle, butényle, pentényle, héxényle.

L'expression alkynyle ayant au plus 6 atomes de carbone inclut les radicaux éthynyle, propargyle, butynyle.

L'expression cycloalkyle ayant au plus 6 atomes de carbone inclut les radicaux cyclopropyle, cyclobutyle, cyclopentyle et cyclohexyle.

Parmi les valeurs de R, on préfère les valeurs $CH_3$, $CHF_2$, $CH_2F$, $CH_2CO_2H$.

Lorsque le radical R représente un radical alkyle, de préférence méthyle, il peut être substitué également par un radical cycloalkyle tel que cyclopropyle. On préfère ainsi le radical

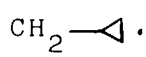

Parmi les valeurs de A, on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) aminométhane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements esters facilement clivables que peut représenter A, les groupements méthoxyméthyle, éthoxyméthyle, isopropyloxyméthyle, alpha-méthoxyéthyle, alpha-éthoxyéthyle, méthylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadénoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtalidyle, tert-butylcarbonylméthyle, allyle, 2-chloroallyle, méthoxycarbonylméthyle, benzyle ou tert-butyle.

On peut encore citer entre autres restes de groupements esters que peut représenter A, les groupements méthoxyéthoxyméthyle, diméthylaminoéthyle, cyanométhyle, tert-butoxycarbonylméthyle, 2,2-éthylidènedioxyéthyle, cyanoéthyle, 2,2-diméthoxyéthyle ; 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoéthoxyméthyle, 2-aminoéthoxyméthyle, 3-méthoxy 2,4-thiadiazol-5-yle, 2-tétrahydropyrannyle, 1-méthoxy 1-méthyléthyl 2-hydroxy 1-méthyléthyle, isopropyle ; carbamoylméthyle, chlorométhyle, 2-chloroéthyle, acétylméthyle, 2-méthylthioéthyle ou thiocyanatométhyle.

On peut encore citer entre autres restes de groupements esters que peut représenter A, les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 1-méthyl 1-acétyloxyéthyle, 1-méthoxyacétyloxyéthyle, 1-acétylcarbonyloxyéthyle, 1-hydroxyacétyloxyéthyle, 1-formylcarbonyloxyéthyle, 1-(2-thiényl) carbonyloxyéthyle, 1-(2-furyl) carbonyloxyéthyle, 1-(5-nitro 2-fyryl) carbonyloxyéthyle, 1-(2-pyrrolyl)carbonyloxyéthyle, 1-(propionyloxycarbonyloxy) éthyle, 1-(propyloxycarbonyloxy) éthyle, 1-(isopropyloxycarbonyloxy) éthyle, 1-(méthoxyéthoxycarbonyloxy) éthyle, 1-allyloxycarbonyloxy) éthyle, 1-[(2,3-époxypropyl) oxycarbonyloxy] éthyle, 1-[(2-furyl) méthyloxycarbonyloxy] éthyle, 1-(2-fluoroéthyl) oxycarbonyloxyéthyle, 1-(méthoxycarbonyloxy) propyle, 1-(méthoxycarbonyloxy) 1-méthyléthyle, (méthoxycarbonyloxy) chlorométhyle, 1-(méthoxycarboxyloxy) 2-chloroéthyle, 1-(méthoxycarbonyloxy) 2-méthoxyéthyle, 1-(méthoxycarbonyloxy) allyle.

Les produits de formule (I) peuvent également se présenter sous forme de sels d'acides organiques ou minéraux.

Parmi les acides avec lesquels on peut salifier le ou les groupements amino des produits (I), on peut citer entre autres, les acides acétique, trifluoroacétique, maléïque, tartrique, méthanesulfonique, benzènesulfonique, para-toluènesulfonique, phosphorique, sulfurique, chlorhydrique, bromydrique, iodhydrique.

Les produits peuvent également se présenter sous forme de sels internes.

L'invention a plus particulèrement pour objet les produits de formule générale (I) telle que définie ci-dessus, dans laquelle R représente un radical alkyle, linéaire ou ramifié, ayant de 1 à 4 atomes de carbone éventuellement substitué par un ou plusieurs radicaux choisis parmi les radicaux carboxy libre, estérifié ou salifié, amino et halogène ainsi que les produits de formule générale (I) dans laquelle $R_1$ représente un radical

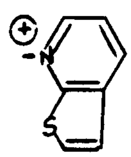

L'invention a plus particulièrement pour objet les produits définis ci-après dans les exemples, à savoir :

- le 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4.2.0.] oct-2-èn-3-yl] 2-propényl] thiéno [2,3-b] pyridinium,
- le 7-[(E)-3-[(6R,7R)-7-[[(2-amino 4-thiazolyl) [(Z)-[(difluorométhoxy) imino]] acétyl] amino] 2-carboxy 8-oxo 5-thia 1-azabicyclo [4.2.0.] oct-2-èn-3-yl] 2-propényl] thiéno [2,3-b] pyridinium,

ainsi que leurs sels avec les métaux alcalin, alcalino-terreux, le magnésium, l'ammoniaque, les bases organiques aminées, les acides, le cas échéant leurs sels internes et leurs esters facilement clivables.

Il est entendu que les produits de formule (I) précités peuvent exister :

- soit sous la forme indiquée par ladite formule (I),
- soit sous la forme de produits de formule ($I_Z$) :

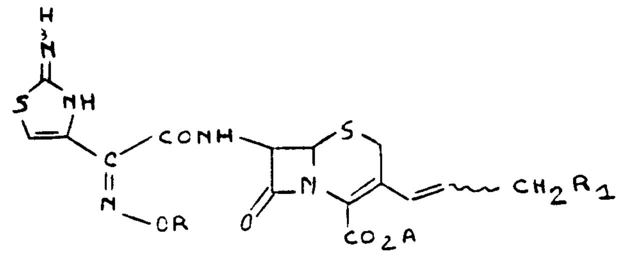

($I_Z$)

dans laquelle R, $R_1$ et A ont la signification précédente.

L'invention concerne également un procédé de préparation des produits de formule (I) telle que décrite ci-dessus, caractérisé en ce que l'on fait agir un réactif choisi parmi les réactifs de formules :

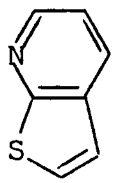 ; 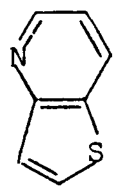 , 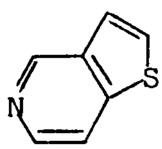 et 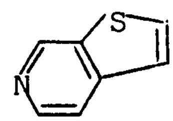

avec un produit de formule (II) :

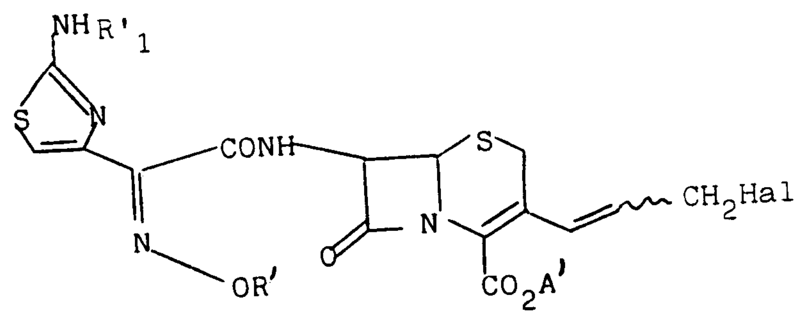

(II)

dans laquelle $R'_1$ représente un atome d'hydrogène ou un groupement protecteur du radical amino, R' représente soit les valeurs indiquées pour R, soit un groupement protecteur du radical hydroxyle, A' représente un atome d'hydrogène ou le reste d'un groupement ester facilement éliminable et Hal représente un atome d'halogène, pour obtenir un produit de formule (III) :

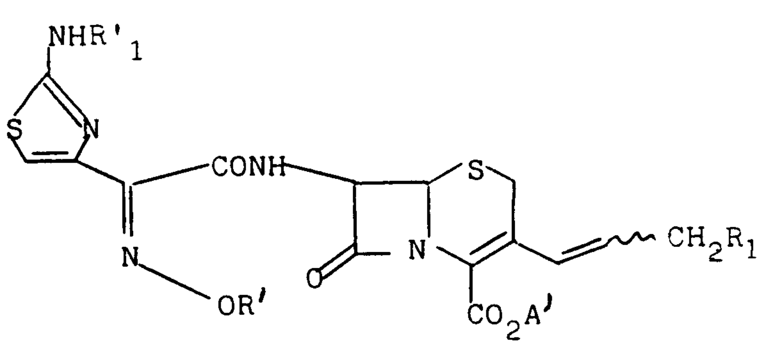

(III)

que, si désiré, l'on sépare en ses isomères E ou Z ou transforme les isomères Z en isomères E et produits de formules (III) que, si nécessaire ou si désiré, l'on soumet à une ou plusieurs des réactions suivantes,

dans un ordre quelconque :

a) coupure par hydrolyse ou par action de la thiourée de tout ou partie des groupements esters ou des groupements de protection du ou des radicaux amino ou du radical hydroxyle,

b) estérification ou salification par une base du ou des radicaux carboxyliques,

c) salification par un acide du ou des radicaux amino.

En plus des groupements cités ci-dessus, le groupement ester facilement éliminable que peut représenter A′ peut être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, 2,2,2-trichloroéthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chlorophényle, tolyle, tert-butylphényle.

Le groupement protecteur du radical amino que peut représenter $R'_1$ peut être par exemple un radical alkyle de 1 à 6 atomes de carbone tel que, préférentiellement, tert-butyle ou tert-amyle. $R'_1$ peut également représenter un groupement acyle aliphatique, aromatique ou hétérocyclique ou un groupe carbamoyle.

On peut citer les groupements alcanoyles inférieurs tel que par exemple formyle, acétyle, propionyle, butyryle, isobutyryle, valéryle, isovaléryle, oxalyle, succinyle, pivaloyle. $R'_1$ peut également représenter un groupe alkoxy ou cycloalkoxycarbonyle inférieur tel que par exemple méthoxycarbonyle, éthoxycarbonyle, propoxycarbonyle, 1-cyclopropyléthoxycarbonyle, isopropyloxycabonyle, butyloxycarbonyle, tert-butyloxycarbonyle, pentyloxycarbonyle hexyloxycarbonyle, un groupe benzoyle, toluolyle, naphtoyle, phtaloyle, mésyle, phénylacétyle, phénylpropionyle, un groupe aralcoxycarbonyle, tel que benzyloxycarbonyle.

Les groupements acyles peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

$R'_1$ peut également représenter un groupement aralkyle inférieur tel que benzyle, 4-méthoxybenzyle, phényléthyle, trityle, 3,4-diméthoxybenzyle ou benzhydryle.

$R'_1$ peut également représenter un groupe haloalkyle tel que trichloroéthyle.

$R'_1$ peut également représenter un groupement chlorobenzoyle, paranitrobenzoyle, para-tert-butylbenzoyle, phénoxyacétyle, caprylyle, n-décanoyle, acryloyle, trichloroéthoxycarbonyle.

$R'_1$ peut également représenter un groupement méthylcarbamoyle, phénylcarbamoyle, naphtylcarbamoyle, ainsi que les thiocarbamoyles correspondants.

La liste ci-dessus n'est pas limitative, il est évident que d'autres groupements protecteurs des amines, groupements connus en particulier dans la chimie des peptides, peuvent également être utilisés.

Le groupement de protection du radical hydroxyle que peut représenter $R'_1$, peut être choisi dans la liste ci-dessous :

R′ peut représenter un groupe acyle tel que par exemple formyle, acétyle, chloroacétyle, bromoacétyle, dichloroacétyle, trichloroacétyle, trifluoroacétyle, méthoxyacétyle, phénoxyacétyle, benzoyle, benzoylformyle, p-nitrobenzoyle. On peut citer également les groupements éthoxycarbonyle, méthoxycarbonyle, propoxycarbonyle, 2,2,2-trichloroéthoxycarbonyle, benzyloxycarbonyle, tert-butoxycarbonyle, 1-cyclopropyléthoxycarbonyle, tétahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxy éthyle, phtaloyle.

On peut également citer d'autres acyles tels que propionyle, butyryle, isobutyryle, valéryle, isovaléryle, oxalyle, succinyle et pivaloyle.

On peut également citer les radicaux phénylacétyle, phénylpropionyle, mésyle, chlorobenzoyle, paranitrobenzoyle, para-tert-butylbenzoyle, caprylyle, acryloyle, méthylcarbamoyle, phénylcarbamoyle, naphtylcarbamoyle.

Dans un mode préférentiel d'exécution du procédé :

- l'action d'un réactif de formule:

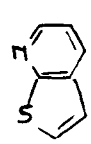 ; 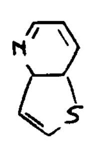 ; 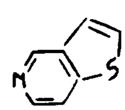 ou 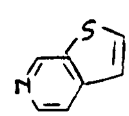

sur le produit de formule (II) est effectuée dans les conditions préférentielles suivantes :

Lorsque Hal représente par exemple un atome de chlore, on effectue in situ ou séparément une substitution de l'atome de chlore par un atome d'iode en présence d'iodure de sodium puis ajoute ensuite le réactif désiré. Des exemples d'une telle réaction sont décrits ci-après dans la partie expérimentale.

Dans la préparation des produits de formule (III), on peut, selon les conditions employées, obtenir un mélange d'isomères E et Z en quantités variables ou au contraire un isomère de façon très prépondérante, le cas échéant par inversion de la configuration du produit de formule (II) lors de la réaction. Lorsque l'on obtient un mélange d'isomères, on peut les séparer par les méthodes usuelles notamment la chromatographie.

Dans les conditions décrites dans la partie expérimentale ci-après, on obtient l'isomère E de façon très prépondérante et seul cet isomère est isolé.

Selon les valeurs de $R'_1$, R′ et A′, les produits de formule (III) peuvent ou non constituer des produits de formule (I).

Les produits de formule (III) constituent des produits de formule (I) lorsque $R'_1$ représente un atome d'hydrogène, lorsque R′ ne représente pas un groupement protecteur du radical hydroxyle que l'on désire éliminer et lorsque A′ ne représente pas, parmi les groupements esters facilement clivables, l'un de ceux que l'on désirerait éliminer.

Dans les autres cas, l'action sur le produit de formule (III) d'un ou plusieurs agents d'hydrolyse, d'hydrogénolyse ou de la thiourée a pour but d'éliminer le radical $R'_1$ lorsque celui-ci représente un radical protecteur du radical amino, d'éliminer le radical R′ lorsque celui-di est différent de R et/ou d'éliminer le radical A′ lorsque celui-ci représente, parmi les groupements esters facilement clivables, l'un de ceux que l'on désire éliminer.

Cependant, il est bien entendu possible d'éliminer $R'_1$ sans toucher aux substituants R′ et A′ lorsque ceux-ci doivent être conservés. Il en est ainsi par exemple lorsque A′ représente un groupement ester que l'on souhaite conserver tel qu'un groupement propionyloxyméthyle.

La nature des réactifs à mettre en jeu dans un tel cas est bien connu de l'homme de métier. Des exemples de telles réactions sont donnés plus loin dans la partie expérimentale.

On trouvera par exemple une description des différentes méthodes d'élimination des différents groupements protecteurs dans la demande de brevet français B.F. 2 499 995.

La salification des produits peut être effectuée selon les méthodes usuelles.

La salification peut, par exemple, être obtenue par action sur un produit sous forme acide ou sur un solvat, par exemple, le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique. On peut également faire appel à des sels d'acides organiques.

Comme sels d'acides organiques, on peut mentionner, par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle, comme par exemple, phényle, thiényle, furyle, par un ou plusieurs radicaux hydroxyles ou par on ou plusiers atomes d'halogène tels que fluor, chlore ou brome, préférentiellement chlore, par on ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoïques substitués, de préférence par des radicaux alkyles inférieurs.

Comme exemples de tels acides organiques, on peut mentionner :
les acides formique, acétique, acrylique, butyrique, adipique, isobutyrique, n-caproïque, isocaproïque, chloropropioniques, crotonique, phénylacétique, 2-thiénylacétique, 3-thiénylacétique, 4-éthylphénylacétique, glutarique, l'ester monoéthylique de l'acide adipique, les acides hexanoïque, heptanoïque, décanoïque, oleïque, stéarique, palmitique, 3-hydroxypropionique, 3-méthoxypropionique, 3-méthylthiobutyrique, 4-chlorobutyrique, 4-phénylbutyrique, 3-phénoxybutyrique, 4-éthylbenzoïque, 1-propylbenzoïque.

On utilise cependant de préférence comme sels de sodium, l'acétate de sodium, le 2-éthylhexanoate de sodium ou le diéthylacétate de sodium.

La salification peut également être obtenue par action d'une base organique comme la triéthylamine, la diéthylamine, la triméthylamine, la propylamine, la N,N-diméthyléthanolamine, le tris (hydroxyméthyl) amino méthane, la méthylamine, l'éthanolamine, la pyridine, la picoline, la dicyclohexylamine, la morpholine et la benzylamine.

Elle peut également être obtenue par action de l'arginine, de la lysine, de la procaïne, de l'histidine, de la N-méthylglucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectué dans les conditions usuelles.

L'estérification éventuelle des produits est effectuée dans les conditions classiques. On opère en général en faisant réagir l'acide de formule (I) ou un dérivé fonctionnel avec un dérivé de formule :

Z-Re

dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou un éventuel groupement oxyimino sont bloqués avant d'enlever le groupement protecteur de l'amine et du groupement oxyimino.

Les produits de formule générale (I) possèdent une très bonne activité antibiotique sur les bactéries gram (+) telles que les staphylocoques, les streptocoques et notamment sur les staphylocoques pénicillino-résistants. Leur efficacité sur les bactéries gram (-) notamment sur les bactéries coliformes, les klebsellia, les salmonella et les proteus est particulièrement remarquable.

Ces propriétés rendent aptes lesdits produits ainsi que leurs sels d'acides pharmaceutiquement acceptables, à être utilisés comme médicaments dans le traitement des affections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aigües primitives ou postgrippales, bronchopneumonies, suppurations pulmonaires.

Ces produits peuvent également être utilisés comme médicaments dans le traitement des colibacilloses et infections associées, dans les infections à proteus, à klebsellia et à salmonella et dans d'autres affections provoquées par des bactéries à gram (-).

La présente invention a donc également pour objet, à titre de médicaments et notamment de médicaments antibiotiques, les produits de formule (I) tels que définis ci-dessus ainsi que leurs sels d'acides pharmaceutiquement acceptables.

L'invention a plus particulièrement pour objet, à titre de médicaments, les produits de formule (I) telle que décrite ci-dessus dans laquelle R représente un radical alkyle, linéaire ou ramifié, ayant de 1 à 4 atomes de carbone éventuellement substitué par un ou plusieurs radicaux choisis parmi les radicaux carboxy libre, estérifié ou salifié, amino ou halogène et ceux dans laquelle $R_1$ représente un radical

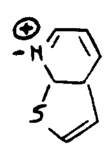

L'invention a spécialement pour objet, à titre de médicaments et notamment de médicaments antibiotiques, les produits décrits ci-après dans les exemples, à savoir :

- le 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4.2.0.] oct-2-èn-3-yl] 2-propényl] thiéno [2,3-b] pyridinium,
- le 7-[(E)-3-[(6R,7R)-7-[[(2-amino 4-thiazolyl) [(Z)-[(difluorométhoxy) imino]] acétyl] amino] 2-carboxy 8-oxo 5-thia 1-azabicyclo [4.2.0.] oct-2-èn-3-yl] 2-propényl] thiéno [2,3-b] pyridinium,

ainsi que leurs sels avec les métaux alcalin, alcalino-terreux, le magnésium, l'ammoniaque, les bases organiques aminées, les acides, le cas échéant leurs sels internes et leurs esters facilement clivables.

L'invention s'étend aux compositions pharmaceutiques renfermant, comme princpe actif, au moins un des médicaments définis ci-dessus.

Ces compositions peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants dispersants ou émulsifiants, les conservateurs.

Ces compositions peuvent notamment se présenter sous forme d'une poudre destinée à être dissoute extemporanément dans un véhiclue approprié, par exemple, de l'eau stérile apyrogène.

La dose administrée est variable selon l'affection traitée, le sujet en cause, la voie d'administration et le produit considéré. Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

L'invention a enfin pour objet, à titre de produits industriels nouveaux, les produits de formule (III) telle que définie ci-dessus dans laquelle $R'_1$ représente un groupement protecteur du radical amino.

En plus des produits décrits dans les exemples qui illustrent l'invention sans toutefois la limiter, les produits suivants constituent des produits pouvant être obtenus dans le cadre de la présente invention ; les substituants $R_1$, A et R sont ceux indiqués dans la formule (I) :

| $R_1$ | A | R | Isomérie 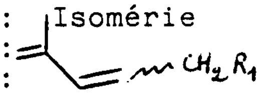 |
|---|---|---|---|
| 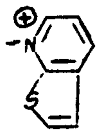 | ⊖ | $CH_2CO_2H$ | E |
| 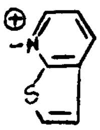 | ⊖ | $CH_3$<br>$- C-CO_2H$<br>$CH_3$ | E |
| 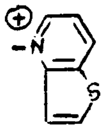 | ⊖ | $CH_3$ | E |
| 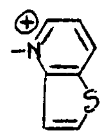 | ⊖ | $CHF_2$ | E |
| 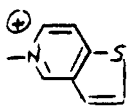 | ⊖ | $CH_3$ | E |
| 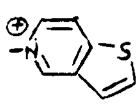 | ⊖ | $CHF_2$ | E |
| 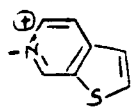 | ⊖ | $CH_3$ | E |
| 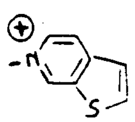 | ⊖ | $CHF_2$ | E |

Les produits de formule (II) sont connus dans la littérature, notamment dans la demande de brevet britannique GB 2 134 522 ou peuvent être préparés selon les méthodes usuelles. Des méthodes de préparation de tels produits sont décrits ci-après dans la partie expérimentale.

Les exemples suivants illustrent l'invention sans toutefois la limiter.

**Exemple 1 : Iodure de 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4.2.0.] oct-2-èn-3-yl] 2-propényl] thiéno [2,3-b] pyridinium.**

**Stade A :**

Iodure de 7-[(E)-3-[(6R,7R)-2-[(diphénylméthoxy) carbonyl] 7-[[[(Z)-(méthoxyimino)] [2-[(triphénylméthyl) amino] 4-thiazolyl] acétyl] amino] 8-oxo 5-thia 1-azabicyclo [4.2.0.] oct-2-èn-3-yl] 2-propényl] thiéno [2,3-b] pyridinium.

On agite 1 heure 10 minutes, 1,033 g de (6R,7R)-3-[(Z)-3-chloro 1-propényl] 7-[[[(Z)-(méthoxyimino)] [2-[(triphénylméthyl) amino] 4-thiazolyl] acétyl] amino] 8-oxo 5-thia 1-azabicyclo [4.2.0.] oct-2-èn-2-carboxylate de diphénylméthyle, 542 mg d'iodure de potassium dans 22 cm3 d'acétone anhydre, évapore le solvant et reprend le résidu dans l'acétate d'éthyle. On lave la phase organique avec du thiosulfate de sodium 0,2N, sèche et évapore le solvant. On ajoute au résidu 334 ul de thiéno [2,3-b] pyridine et 3,2 cm3 de diméthylsulfoxyde anhydre, agite 1 heure 15 minutes, verse le milieu réactionnel dans l'eau et extrait à l'acétate d'éthyle. On lave à l'eau les phases organiques, sèche et évapore à sec. On chromatographie sur silice le résidu, élue par les mélanges chlorure de méthylène-méthanol (95-5) puis (92-8) et obtient 259 mg de produit attendu.

<u>Spectre RMN</u> ($CDCl_3$ 250 MHz ppm) :

| | | |
|---|---|---|
| 3,70 | : | $CH_2S$ |
| 4,05 | : | OMe |
| 5,07 | | $H_6$ } cis |
| 5,95 | | $H_7$ } |
| 5,65 | : | $CH_2N^+$ |
| 6,58 et 7,19 | } | éthynéliques E Δ J ≃ 16 Hz |
| 6,70 | : | $H_5$ du thiazole |
| 6,98 | : | COO-CH |
| 7,02-7,04 | : | aromatiques |
| 6,87 et 7,0 | : | NH |
| 7,66 | : | $H_3$ |
| 8,81 | : | $H_2$ |
| 8,03 | : | $H_5$ hétérocycles condensés |
| 8,81 | : | $H_4$ |
| 9,96 | : | $H_6$ |

**<u>Stade B</u> :**

Iodure de 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4.2.0.] oct-2-èn-3-yl] 2-propényl] thiéno [2,3-b] pyridinium.

On dissout 150 mg de produit obtenu ci-dessus dans 2 cm3 d'acide formique à 66%, chauffe à 70¤C pendant 45 minutes. On évapore le solvant sous pression réduite, empâte le résidu à l'éther, filtre, rince à l'éther et obtient 94 mg de produit attendu. On reprend 51 mg de produit ainsi obtenu dans 0,25 cm3 d'acide formique à 66% et chauffe à 60¤C pendant 1 heure 30 minutes. On refroidit, ajoute 0,25 cm3 d'eau déminéralisée et lave à l'éther éthylique. On évapore la solution aqueuse à sec et reprend à plusieurs reprises par de l'éthanol anhydre. On isole les cristaux formés et lave à ' l'éthanol par centrifugation. On obtient 30 mg de produit attendu.

Spectre RMN (DMSO 250 MHz ppm) :

| | | |
|---|---|---|
| 6,73 | : | $H_5$ du thiazole |
| 5,8 | : | $H_7$ (céphème, J=5Hz) |
| 5,2 | : | $H_6$ (J=16Hz = $\Delta$ E) |
| 7,12 | | éthyléniques |
| 6,33 | | éthyléniques |
| 3,67 | : | $CH_2N^+<$ |
| 9,03 | : | en ortho du pyridyle |
| 8,14 | : | en méta du pyridyle (t, J=8Hz) |
| 9,08 | : | en para du pyridyle (d, J=8Hz) |
| 8,28 | : | en alpha du thiényle |
| 7,89 | : | en béta du thiényle (d, J=6Hz) |

**Exemple 2 : (6R,7R)-7-[[(2-amino 4-thiazolyl) [(Z)-[(difluorométhoxy) imino]] acétyl] amino] 8-oxo 3-[-(E)-3-[7-[thiéno [2,3-b] pyridinio] 1-propényl] 5-thia 1-azabicyclo [4.2.0] oct-2-èn-2-carboxylate (sel interne).**

**Stade A :**

(6R,7R)-(3-chlorométhyl) 7-[[[(Z)-(difluorométhoxy) imino] [2-[(triphénylméthyl) amino] 4-thiazolyl] acétyl] amino] 8-oxo 5-thia 1-azabicyclo [4.2.0.] oct-2-èn-2-carboxylate de diphénylméthyle.

On mélange en agitant 948,2 mg de chlorhydrate de (6R,7R) 7-amino 3-(chlorométhyl) 8-oxo 5-thia 1-azabicyclo [4.2.0.] oct-2-èn-2-carboxylate de diphénylméthyle, 1,108 g d'acide [(difluorométhoxy) imino] [2-[(triphénylméthyl) amino] 4-thiazolyl] acétique dans 21 cm3 de chlorure de méthylène et ajoute goutte à goutte 362,3 ul de diisopropylcarbodiimide et maintient l'agitation pendant une demi-heure à température ambiante. On évapore à sec le mélange réactionnel à 30¤C maximum sous pression réduite. On chromatiographie le résidu sur silice avec un mélange chlorure de méthylène-acétate d'éthyle (97-3). On isole 1,506 g de produit attendu.

Spectre RMN ($CDCl_3$ 250 MHz ppm) :

| | | | |
|---|---|---|---|
| 6,86 (s) | : | $H_5$ du thiazole | |
| 6,78 | : | $OC\underline{H}F_2$ (t, J=72Hz) | |
| 5,95 | : | $H_7$ (d, J=5Hz) | du céphème |
| 5,10 | : | $H_6$ (d, J=5Hz) | du céphème |
| 4,40 | : | $-C\underline{H}_2-Cl$ ($J_AB$ = 12Hz) | |
| 6,97 (s) | : | $CO_2C\underline{H}\phi_2$ | |

**Stade B :**

Iodure de [[(6R,7R)-7-[[[((Z)-[(difluorométhoxy) imino]] ]2-[(triphénylméthyl) amino] 4-thiazolyl] acétyl] amino] 2-[(diphénylméthoxy)carbonyl] 8-oxo 5-thia 1-azabicyclo [4.2.0.] oct-2-èn-3-yl] méthyl] triphényl phosphonium.

On agite une heure à température ambiante 1,467 g de produit obtenu ci-dessus dans 20,2 cm3 de méthyléthylcétone anhydre et 1,255 g d'iodure de sodium sec. On amène à sec sous pression réduite à 30¤C maximum, reprend le résidu par 82 cm3 d'acétate d'éthyle, lave la phase organique avec une

solution 0,5N de thiosulfate de sodium puis à l'eau et sèche la solution de dérivé iodo obtenue. A celle-ci, on ajoute en 1 fois 915 mg de triphényl phosphine, agite 20 minutes et amène à sec sous pression réduite. On empâte le résidu à l'éther (60 cm3), agite 1/4 d'heure, essore et obtient 1,74 g de produit attendu.

<u>Spectre RMN</u> ($CDCl_3$ 250 MHz ppm) :

6,49 : $H_5$ du thiazole
6,78 : $OC\underline{H}F_2$ (t, J=72Hz)
5,91 : $H_7$ (d,d J=5 et 5Hz) } du céphème cis
5,07 : $H_6$ (d, J=5Hz) }
4,46 : $-C\underline{H}-P\phi_3$ (d,d J=5,5 et 20Hz)
5,12 : $\underline{H}$ (de $-CH-P\phi_3$)
6,87 : $CO_2C\underline{H}\phi_2$

**<u>Stade C</u> :**

(6R,7R)-3-[(Z)-3-chloro 1-propényl] 7-[[[(Z)-[(difluorométhoxy) imino]] [2-[(triphénylméthyl) amino] 4-thiazolyl] acétyl] amino] 8-oxo 5-thia 1-azabicyclo [4.2.0.] oct-2-èn-2-carboxylate de diphénylméthyle.

On mélange 799,6 mg de produit obtenu en B dans 8,5 cm3 de chloroforme, ajoute 5,8 cm3 d'eau puis 0,78 cm3 de soude N et agite 5 minutes. On décante en présence de chlorure de sodium, extrait les phases aqueuses au chloroforme, réunit les phases organiques, lave à l'eau, sèche et filtre. On ajoute au filtrat du sulfate de magnésium, 0,51 cm3 d'une solution aqueuse à 50-55% de chloroacétaldéhyde et agite 3 heures à température ambiante. On filtre, évapore à sec, chormatographie le résidu sur silice (éluant : chlorure de méthylène-éther éthylique 97-3) et obtient 145 mg de produit attendu.

<u>Spectre RMN</u> ($CDCl_3$ 250 MHz ppm) :

6,89 et 6,93 : $H_5$ du thiazole et $O-C\underline{H}\phi_2$
5,93 $H_7$ (J=5Hz) } du céphème cis
$H_6$ }
5,15 } delta Z (d, J=5Hz)
5,63 } delta Z (m)
6,22 } delta Z (d, J=11Hz)
3,31 à 3,48 : $ClC\underline{H_2}-CH$ et $S-C\underline{H_2}$
de 7,25 à 7,35 : les phényles.

**<u>Stade D</u> :**

Iodure de 7-[(E)-3-[(6R,7R)-7-[[[(Z)-[(difluorométhoxy) imino]] [2-[(triphénylméthyl) amino] 4-thiazolyl] acétyl] amino] 2-[(diphénylméthoxy) carbonyl] 8-oxo 5-thia 1-azabicyclo [4.2.0.] oct-2-èn-3-yl] 2-propényl] thiéno [2,3-b] pyridinium.

On opère comme au stade B ci-dessus à partir de 303 mg du produit obenu au stade C dans 5 cm3 d'acétone anhydre et 252 mg d'iodure de potassium et obtient 327 mg de dérivé iodé. On ajoute à celui-ci 227 mg de thiéno [2,3-b] pyridine et 2,5 cm3 d'acétonitrile anhydre puis agite une heure à température ambiante, enfin on évapore à sec. On chromatographie le résidu sur silice, élue d'abord avec un mélange chlorure de méthylène-éther éthylique (9-1) puis chlorure de méthylène-méthanol (95-5) et isole 95 mg de produit attendu.

Spectre RMN ($CDCl_3$ 60 MHz ppm) :

| | | | | |
|---|---|---|---|---|
| 6,84 et 6,97 | : | $H_5$ du thiazole et $-C\underline{H}\phi_2$ | | |
| 6,75 | : | $-OC\underline{H}F_2$ | | (t , J=72Hz) |
| 5,92 | | $H_7$ | du cèphème cis | |
| 5,10 | | $H_6$ | du cèphème cis | |
| 6,6 | : | $=CH-CH_2$ | | (d,t J=7 et 15Hz) |
| 5,66 | : | $-C\underline{H_2}N^+$ | | |
| 7,66 | : | $H_4$ du thiènyle | cycle thiéno [2,3-b] pyridinium | (d, J=6Hz) |
| 7,81 | : | $H_5$ du thiènyle | cycle thiéno [2,3-b] pyridinium | (d, J=6Hz) |
| 8,78 | : | H en gamma de $N^+$ | cycle thiéno [2,3-b] pyridinium | (d, J=8Hz) |
| 8,02 | : | H en bêta de $N^+$ | cycle thiéno [2,3-b] pyridinium | (m) |
| 9,94 | : | H en alpha de $N^+$ | cycle thiéno [2,3-b] pyridinium | (d, J=6Hz) |

**Stade E :**

(6R,7R)-7-[[(2-amino 4-thiazolyl) [(Z)-[difluorométhoxy) imino]] acétyl] amino] 8-oxo 3-[(E)-3-[7-[thiéno [2,3-b] pyridinio] 1-propényl] 5-thia 1-azabicyclo [4.2.0.] oct-2-èn-2-carboxylate.

On agite 1/4 d'heure 110 mg de produit obtenu ci-dessus avec 3,5 cm3 d'acide trifluoroacétique à 10% d'anisole, filtre l'insoluble en faisant couler le filtrat goutte à goutte directement dans 35 cm3 d'éther isopropylique vivement agités. On centrifuge, effectue deux lavages à l'éther isopropylique en empâtant le résidu et centrifugeant à chaque fois. On sèche sous pression réduite et obtient 67 mg de produit attendu. On effectue un HPLC sur 60 mg de produit (colonne lichrosorb RP 18) avec de l'eau à 20% d'acétonitrile. Après lyophilisation, on obtient 22 mg de produit.

Spectre RMN (DMSO 250 MHz ppm) :

| | | | | |
|---|---|---|---|---|
| 6,99 | : | $H_5$ du thiazole | | |
| 7,10 | : | $-OCHF_2$ | | (t , J=72Hz) |
| 5,64 | : | $H_7$ | du céphème | (d après échange) |
| 5,09 | : | $H_6$ | du céphème | |
| 7,4 | : | $-CH=CH-$delta E | | (d,t J=15Hz) |
| 5,91 | : | | | (d, t J=15 et 7Hz) |
| 9,22 | : | H en alpha de $N^+$ | cycle thiéno [2,3-b] pyridinium | (d) |
| 8,13 | : | H en bêta de $N^+$ | cycle thiéno [2,3-b] pyridinium | (t) |
| 9,05 | : | H en gamma de $N^+$ | cycle thiéno [2,3-b] pyridinium | (d) |
| 7,85 | : | H en alpha de S | cycle thiéno [2,3-b] pyridinium | (d) |
| 8,28 | : | H en bêta de S | cycle thiéno [2,3-b] pyridinium | (d) |

**Exemple 3 : (6R,7R)-7-[[(2-amino 4-thiazolyl) [(Z)[méthoxyimino]] acétyl] amino] 8-oxo 3-[(E)-3-[7-[thiéno [2,3-b] pyridinio] 1-propényl] 5-thia 1-azabicyclo [4.2.0.] oct-2-èn-2-carboxylate (sel interne).**

**Stade A :**

Tétrafluoroborate de 7-[(E)-3-[(6R,7R)-2-[(diphénylméthoxy) carbonyl] 7-[[[(Z)-(méthoxyimino)] [2-[-(triphénylméthyl) amino ] 4-thiazolyl] acétyl] amino] 8-oxo 5-thia 1-azabicyclo [4.2.0.] oct-2-èn-3-yl] 2-propényl] thiéno [2,3-b] pyridinium.

On mélange 1,331 g de tétrafluoroborate d'argent et 40 cm3 de chlorure de méthylène puis ajoute le mélange constitué de 3,366 g de (6R,7R)-3-[(Z)-3-chloro 1-propényl] 7-[[[(Z)-(méthoxyimino)] ]2-[-(triphénylméthyl) amino] 4-thiazolyl] acétyl] amino] 8-oxo 5-thia 1-azabicyclo [4.2.0.] oct-2-èn-2-carboxylate de diphénylméthyle, 38 cm3 de chlorure de méthylène et 1,05 cm3 de thiéno [2,3-b] pyridine. On mélange pendant 3 heures et 20 minutes à 20¤C, filtre, évapore le solvant et chromatographie sur silice en éluant au chlorure de méthylène puis avec des mélanges chlorure de méthylène-méthanol renfermant 2, 4, 6 et 8% de méthanol. On obtient finalement 2,004 g de produit attendu.

Spectre RMN ($CDCl_3$ 300 MHz ppm) :

| | | | |
|---|---|---|---|
| 3,67 | : | $SCH_2$-C=C | |
| 4,03 | : | N-O-$CH_3$ | (s) |
| 5,03 | : | $H_6$ | (d, J=5Hz) |
| 5,93 | : | $H_7$ (cis) | (dd, J=5Hz) |
| 5,33 | : | $N^+CH_2$-CH= | (d, J=7Hz) |
| 6,27 | : | $N^+CH_2$-CH= | (td, J=7 et 16Hz) |
| 7,16 | : | =C-CH=CH-$CH_2N^+$ | (d, J=16Hz) |
| 6,97 | : | $CO_2CHPh_2$ | (s) |
| 6,69 | : | $H_5$ du thiazole | (s) |
| 6,89 | : | NHCH | (d) |
| 7,04 | : | $NHCPh_3$ | (s) |
| 7,2 à 7,4 | : | $CPh_3$ et $CHCPh_2$ | (m) |
| 7,62 | : | $H_3$ du thiénopyridinium | (d, J=6Hz) |
| 7,74 | : | $H_2$ du thiénopyridinium | (d, J=6Hz) |
| 7,93 | : | $H_5$ du thiénopyridinium | (dd, J=6 et 8Hz) |
| 8,74 | : | $H_4$ du thiénopyridinium | (d, J=8Hz) |
| 9,10 | : | $H_6$ du thiénopyridinium | (d, J=8Hz) |

**Stade B :**

(6R,7R)-7-[[(2-amino 4-thiazolyl) [(Z)[méthoxyimino]] acétyl] amino] 8-oxo 3-[(E)-3-[7-[thiéno [2,3-b] pyridinio] 1-propényl] 5-thia 1-azabicyclo [4.2.0.] oct-2-èn-2-carboxylate (sel interne).

On dissout le produit obtenu au stade A dans 27 cm3 d'acide formique à 33% d'eau, place dans un bain à 70¤C pendant 1 heure puis évapore à sec. On reprend le résidu par un mélange éthanol-eau puis évapore à sec de nouveau. On empâte le résidu à l'éther et filtre. On dissout le produit dans 2,5 cm3 d'une solution aqueuse 1M de carbonate de triéthylamine et 2,5 cm3 d'acétonitrile. On effectue une HPLC sur le produit (colonne lichrosorb RP 18) en éluant avec des mélanges acétonitrile-eau (5-95), (10-90), (15-85) et

(20-80). On isole sous forme liophylisée 0,652 g de produit attendu.

Spectre RMN (DMSO 300 MHz ppm) :

| | | | |
|---|---|---|---|
| 3,82 | : | N-O-$CH_3$C | (s) |
| 5,04 | : | $H_6$ | (d, J=4,5Hz) |
| 5,59 | : | $H_7$ (cis) | (m) |
| 5,58 | : | $N^+CH_2$-CH= | |
| 5,87 | : | $N^+CH_2$-CH- | (t d) |
| 7,38 | : | =C-CH=CH-$CH_2N^+$ | (d, J=17Hz) |
| 6,73 | : | $H_5$ du thiazole | (s) |
| 9,37 | : | NHCH | (d) |
| 9,23 | : | $H_3$ du thiénopyridinium | (d) |
| 9,06 | : | $H_5$ du thiénopyridinium | (d) |
| 8,12 | : | $H_4$ du thiénopyridinium | (t) |
| 7,87 | : | $H_6$ du thiénopyridinium | (d) |
| 8,28 | : | $H_7$ du thiénopyridinium | (d) |
| 7,23 | : | $NH_2$ | |

**Exemple 4 :**

On a réalisé des préparations pour injections de formule :

| | |
|---|---|
| - 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4.2.0.] oct-2-èn-3-yl] 2-propényl] thiéno [2,3-b] pyridinium | 500 mg |
| - Excipient aqueux stérile q.s.p. | 5 cm3. |

**ETUDE PHARMACOLOGIOUE DES PRODUITS DE L'INVENTION.**

**Activité in vitro, méthode des dilutions en milieu liquide.**

On prépare une série de tubes dans lequel on répartit une même quantité de milieu nutritif stérile. On distribue dans chaque tube des quantités croissantes du produit a étudier, puis chaque tube est ensemencé avec une souche bactérienne. Après incubation de 24 ou 48 heures à l'étuve à 37¤C, l'inhibition de la croissance est appréciée par transillumination, ce qui permet de déterminer les concentrations minimales inhibitrices (C.M.I.) exprimées en microgrammes/cm3.

Les résultats suivants sont obtenus :

| SOUCHES | CMI EN µG/ML Produit de l'ex. 1 24 H | Produit de l'ex. 2 24 H | Produit de l'ex. 3 24 H | Produit de l'ex. 4 24 H |
|---|---|---|---|---|
| Staphylococcus aureus 285 | 0,15 | 5 | 2,5 | 0,15 |
| Staphylococcus aureus Exp. n° 54 146 | 0,3 | 5 | 2,5 | 0,3 |
| Streptococcus pyogenes A 561 | ≤ 0,01 | ≤ 0,01 | ≤ 0,01 | ≤ 0,01 |
| Streptococcus pyogens 77 A | ≤ 0.01 | ≤ 0,01 | ≤ 0.01 | ≤ 0,01 |
| Escherichia Coli UC 1894 | ≤ 0,01 | 0,04 | 0,08 | ≤ 0,01 |
| Escherichia Coli O 78 | 0,02 | 1,2 | 0,3 | 0,02 |
| Escherichia Coli T E M | 0,04 | 0,6 | 0,6 | 0,08 |
| Escherichia Coli 1507 E | 0,01 | 0,15 | 0,04 | 0,01 |
| Escherichia Coli DC O | 0,04 | 2,5 | 1,2 | 0,04 |
| Escherichia coli DC 2 | 0,01 | 0,6 | 0,08 | 0,01 |
| Salmonella typhimurium MZ 11 | 0,04 | 0,3 | 0,3 | 0,04 |
| Klebsiella pneumoniae Exp. 52 145 | 0,08 | 0,6 | 1,2 | 0,15 |
| Klebsiella aerogenes 1 522 E | 0,15 | 0,6 | 0,6 | 0,08 |
| Enterobacter cloacae 1 321 E | 0,04 | 0,3 | 0,6 | 0,04 |
| Serratia RG 2 532 | 0,15 | 10 | 5 | 0,15 |
| Proteus mirabilis (indol-) A 235 | 0,04 | 0,08 | 0,04 | 0,02 |
| Proteus vulgaris (indol) A 232 | 0,3 | 0,3 | 0,2 | 0,04 |
| Providencia Du 48 | 0,3 | 2,5 | 1,2 | 0,6 |
| Pseudomonas aeruginosa 1771 m | 0,6 | 1,2 | 2,5 | 0,3 |

## Revendications

### Revendications pour les Etats contractants suivants : AT, BE, CH, DE, FR, GB, IT, LI, LU, NL, SE

**1.** Les produits de formule générale (I) :

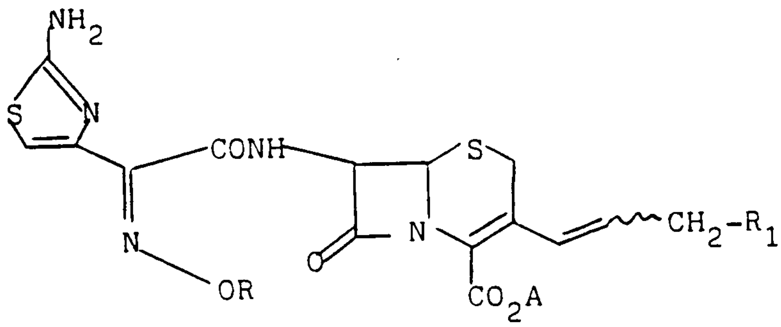

(I)

isomère <u>syn</u>

dans laquelle R représente un atome d'hydrogène ou un radical alkyle, linéaire ou ramifié, alkényle, alkynyle ou cycloalkyle ayant au plus 6 atomes de carbone, chacun de ces radicaux étant éventuellement substitué par un ou plusieurs des radicaux suivants :

carboxy éventuellement salifié ou estérifié, méthoxycarbonyle, éthoxycarbonyle, carbamoyle, diméthylcarbamoyle ; amino ; diméthylamino ; méthylamino ; halogène ; méthoxy, éthoxy, propyloxy ; méthylthio, éthylthio ; phényle ; tétrazolyle ; phénylthio ; tétrazolylthio, thiadiazolylthio éventuellement substitué par méthyle ; $R_1$ représente un radical choisi parmi les radicaux suivants :

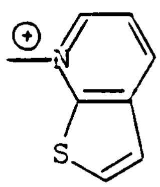 ; 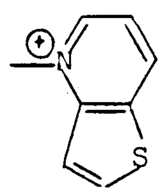 , 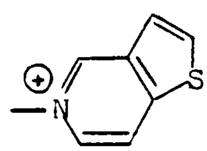 et 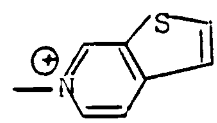

A représente un atome d'hydrogène, un équivalent de métal alcalin, alcalino-terreux, de magnésium, d'ammonium ou d'une base organique aminée ou A représente le reste d'un groupement ester facilement clivable ou $CO_2A$ représente $CO_2^{\ominus}$ ; le trait ondulé signifie que le groupement $CH_2R_1$ peut se trouver dans la position E ou Z ainsi que les sels des produits de formule (I) avec les acides minéraux ou organiques.

**2.** Les produits de formule générale (I) telle que définie à la revendication 1, dans laquelle R représente un radical alkyle, linéaire ou ramifié, ayant de 1 à 4 atomes de carbone éventuellement susbtitué par un ou plusieurs radicaux choisis parmi les radicaux carboxylique libre, estérifié ou salifié, amino ou halogène.

**3.** Les produits de formule générale (I) telle que définie à la revendication 1 ou 2, dans laquelle $R_1$ représente un radical

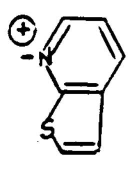

**4.** L'un quelconque des produits de formule (I) selon la revendication 1, dont les noms suivent :

- le 7-[(E)-3-[(6R,7R)-7-[[(2-amino 4-thiazolyl) [(Z)-(méthoxyimino)] acétyl] amino] 2-carboxy 8-oxo 5-thia 1-azabicyclo[4,2,O]oct-2-èn-3-yl] 2-propényl] thiéno[2,3-b] pyridinium,
- le 7-[(E)-3-[(6R,7R)-7[[(2-amino 4-thiazolyl) [(Z)-[(difluorométhoxy) imino]] acétyl] amino] 2-carboxy 8-oxo 5-thia 1-azabicyclo [4.2.0] oct-2-èn-3-yl] 2-propényl] thiéno [2,3-b] pyridinium,

ainsi que leurs sels avec les métaux alcalin, alcalino-terreux, le magnésium, l'ammoniaque, les bases organiques aminées, les acides, le cas échéant leurs sels internes et leurs esters facilement clivables.

**5.** Procédé de préparation des produits de formule (I) telle que définie à la revendication 1, caractérisé en ce que l'on fait agir un réactif choisi parmi les réactifs de formules :

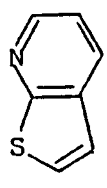 ; 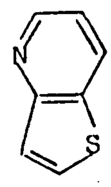 , 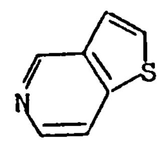 et 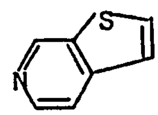

avec un produit de formule (II) :

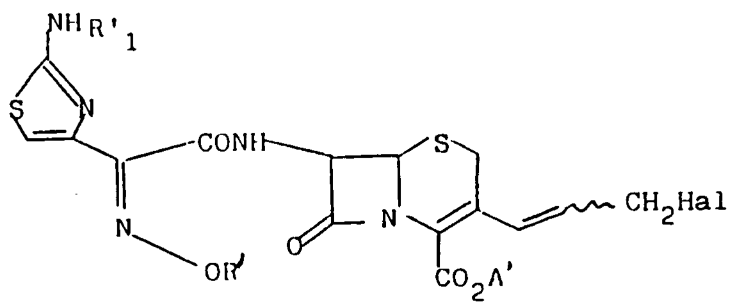

(II)

dans laquelle $R'_1$ représente un atome d'hydrogène ou un groupement protecteur du radical amino, R' représente soit les valeurs indiquées à la revendication 1 pour R, soit un groupement protecteur du radical hydroxyle, A' représente un atome d'hydrogène ou le reste d'un groupement ester facilement éliminable et Hal représente un atome d'halogène, pour obtenir un produit de formule (III) :

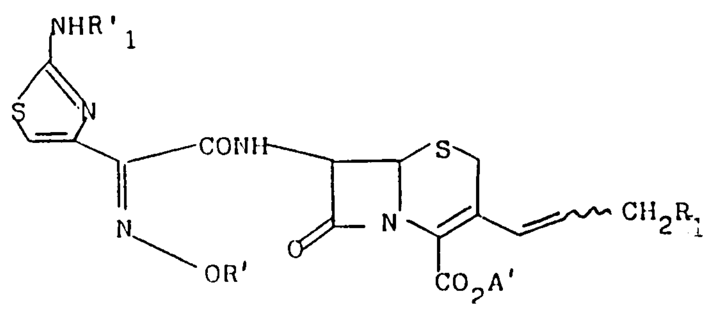

(III)

que, si désiré, l'on sépare en ses isomères E ou Z ou transforme les isomères Z en isomères E et produits de formules (III) que, si nécessaire ou si désiré, l'on soumet à une ou plusieurs des réactions suivantes, dans un ordre quelconque :

a) coupure par hydrolyse ou par action de la thiourée de tout ou partie des groupements esters ou des groupements de protection du ou des radicaux amino ou du radical hydroxyle,

b) estérification ou salification par une base du ou des radicaux carboxyliques,

c) salification par un acide du ou des radicaux amino.

6. A titre de médicaments, les produits répondant à la formule (I) telle que définie à la revendication 1, ainsi que leurs sels d'acides pharmaceutiquement acceptables.

7. A titre de médicaments, les produits tels que définis à l'une quelconque des revendications 2 à 4, ainsi que leurs sels d'acides pharmaceutiquement acceptables.

8. Compositions pharmaceutiques contenant, à titre de principe actif, au moins un médicament selon l'une des revendications 6 ou 7.

9. A titre de produits industriels nouveaux, les produits de formule (III) telle que définie à la revendication 5, dans laquelle $R'_1$ représente un groupement protecteur du radical amino.

**Revendications pour l'Etat contractant suivant : ES**

1. Procédé de préparation des produits de formule générale (I) :

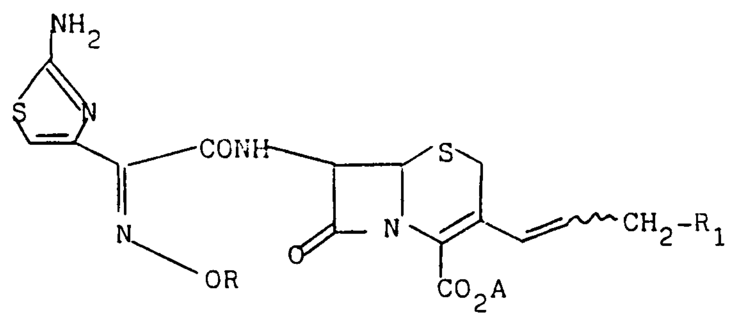

(I)

isomère syn
dans laquelle R représente un atome d'hydrogène ou un radical alkyle, linéaire ou ramifié, alkényle, alkynyle ou cycloalkyle ayant au plus 6 atomes de carbone, chacun de ces radicaux étant éventuellement substitué par un ou plusieurs des radicaux suivants :
carboxy éventuellement salifié ou estérifié, méthoxycarbonyle, éthoxycarbonyle, carbamoyle, diméthylcarbamoyle ; amino ; diméthylamino ; méthylamino ; halogène ; méthoxy, éthoxy, propyloxy ; méthylthio, éthylthio ; phényle ; tétrazolyle ; phénylthio ; tétrazolylthio, thiadiazolylthio éventuellement substitué par méthyle ;
$R_1$ représente un radical choisi parmi les radicaux suivants :

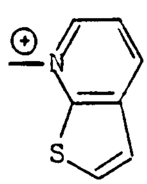 ; 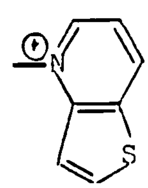 , 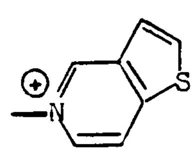 et 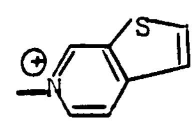

A représente un atome d'hydrogène, un équivalent de métal alcalin, alcalino-terreux, de magnésium, d'ammonium ou d'une base organique aminée ou A représente le reste d'un groupement ester facilement clivable ou $CO_2A$ représente $CO_2^-$, le trait ondulé signifie que le groupement $CH_2R_1$ peut se trouver dans la position E ou Z, ainsi que des sels des produits de formule (I) avec les acides minéraux ou organiques, caractérisé en ce que l'on fait agir un réactif choisi parmi les réactifs de formules :

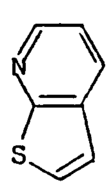 ; 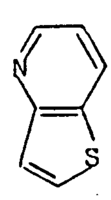 , 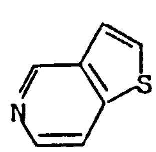 et 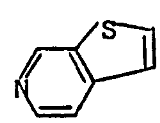

avec un produit de formule (II) :

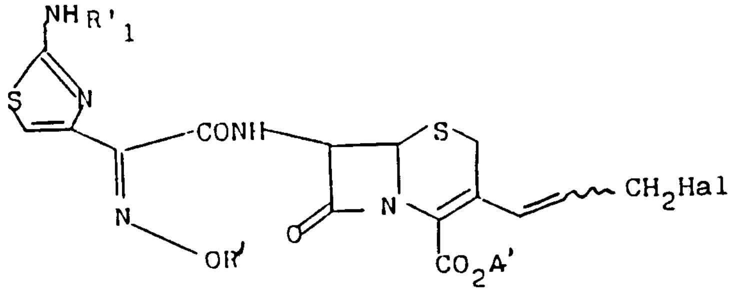

(II)

dans laquelle R'$_1$ représente un atome d'hydrogène ou un groupement protecteur du radical amino, R' représente soit les valeurs indiquées précédemment, soit un groupement protecteur du radical hydroxyle, A' représente un atome d'hydrogène ou le reste d'un groupement ester facilement éliminable et Hal représente un atome d'halogène, pour obtenir un produit de formule (III) :

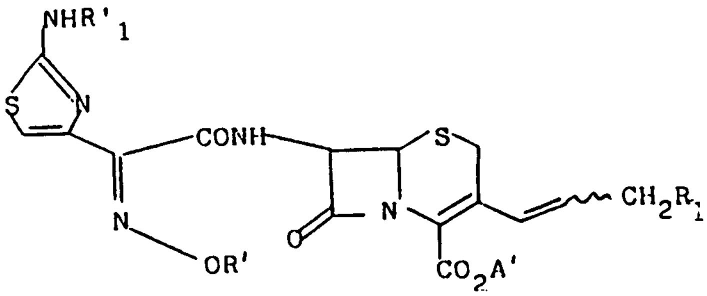

(III)

que, si désiré, l'on sépare en ses isomères E ou Z ou transforme les isomères Z en isomères E et produits de formules (III) que, si nécessaire ou si désiré, l'ou soumet à une ou plusieurs des réactions suivantes, dans un ordre quelconque :

a) coupure par hydrolyse ou par action de la thiourée de tout ou partie des groupements esters ou des groupements de protection du ou des radicaux amino ou du radical hydroxyle,
b) estérification ou salification par une base du ou des radicaux carboxyliques,
c) salification par un acide du ou des radicaux amino.

**2.** Procédé selon la revendication 1, caractérisé en ce que l'on utilise au départ un produit de formule (II) dans laquelle R' représente un radical alkyl linéaire ou ramifié, ayant de 1 à 4 atomes de carbone, éventuellement substitué par un ou plusieurs radicaux choisis parmi les radicaux carboxy libre ou estérifié, amino ou halogène.

**3.** Procédé selon la revendication 1 ou 2, caractérisé en ce que l'on utilise au départ un réactif de formule :

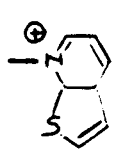

**4.** Procédé selon la revendication 1, 2 ou 3, caractérisé en ce que l'on prépare l'un quelconque des produits de formule (I) selon la revendication 1, dont les noms suivent :

- le 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4.2.0.] oct-2-èn-3-yl] 2-propényl] thiéno [2,3-b] pyridinium,
- le 7-[(E)-3-[(6R,7R)-7-[[(2-amino 4-thiazolyl) [(Z)-[(difluorométhoxy) imino]] acétyl] amino] 2-carboxy 8-oxo 5-thia 1-azabicyclo [4.2.0.] oct-2-èn-3-yl] 2-propényl] thiéno [2,3-b] pyridinium,

ainsi que leurs sels avec les métaux alcalin, alcalino-terreux, le magnésium, l'ammoniaque, les bases organiques aminées, les acides, le cas échéant leurs sels internes et leurs esters facilement clivables.

**Revendications pour l'Etat contractant suivant : GR**

1. Procédé de préparation des produits de formule générale (I) :

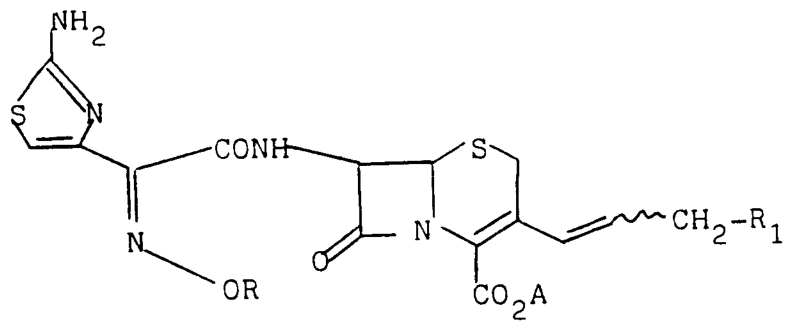

(I)

isomère syn

dans laquelle R représente un atome d'hydrogène ou un radical alkyle, linéaire ou ramifié, alkényle, alkynyle ou cycloalkyle ayant au plus 6 atomes de carbone, chacun de ces radicaux étant éventuellement substitué par un ou plusieurs des radicaux suivants :

carboxy éventuellement salifié ou estérifié, méthoxycarbonyle, éthoxycarbonyle, carbamoyle, diméthylcarbamoyle ; amino ; diméthylamino ; méthylamino ; halogène ; méthoxy, éthoxy, propyloxy ; méthylthio, éthylthio ; phényle ; tétrazolyle ; phénylthio ; tétrazolylthio, thiadiazolylthio éventuellement substitué par méthyle ;

$R_1$ représente un radical choisi parmi les radicaux suivants :

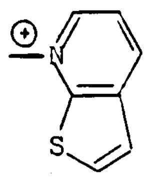 ; 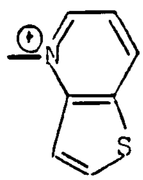 , 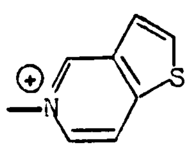 et 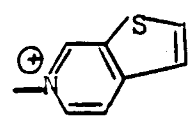

A représente un atome d'hydrogène, un équivalent de métal alcalin, alcalino-terreux, de magnésium, d'ammonium ou d'une base organique aminée ou A représente le reste d'un groupement ester facilement clivable ou $CO_2A$ représente $CO_2^-$, le trait ondulé signifie que le groupement $CH_2R_1$ peut se trouver dans la position E ou Z, ainsi que des sels des produits de formule (I) avec les acides minéraux ou organiques, caractérisé en ce que l'on fait agir un réactif choisi parmi les réactifs de formules :

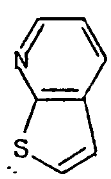 ; 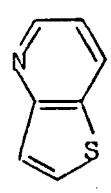 , 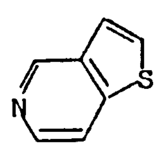 et 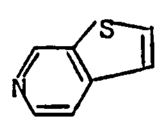

avec un produit de formule (II) :

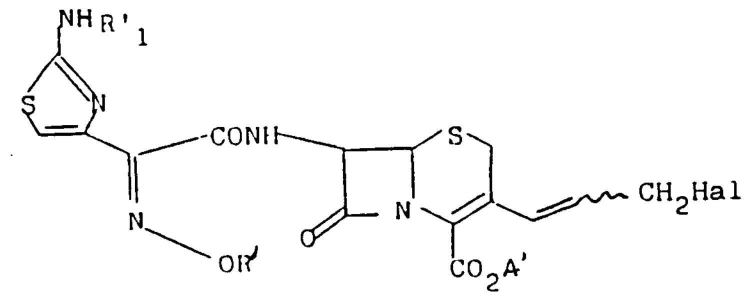

(II)

dans laquelle R'$_1$ représente un atome d'hydrogène ou un groupement protecteur du radical amino, R' représente soit les valeurs indiquées précédemment, soit un groupement protecteur du radical hydroxyle, A' représente un atome d'hydrogène ou le reste d'un groupement ester facilement éliminable et Hal représente un atome d'halogène, pour obtenir un produit de formule (III) :

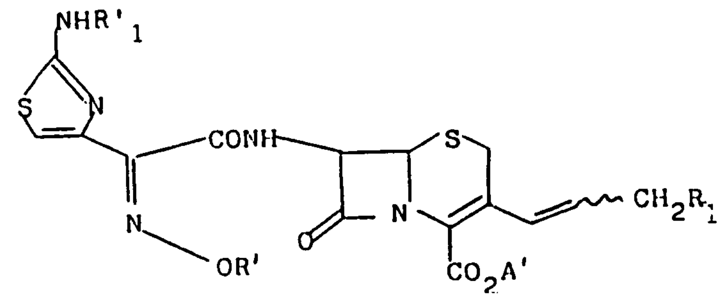

(III)

que, si désiré, l'on sépare en ses isomères E ou Z ou transforme les isomères Z en isomères E et produits de formules (III) que, si nécessaire ou si désiré, l'on soumet à une ou plusieurs des réactions suivantes, dans un ordre quelconque :

a) coupure par hydrolyse ou par action de la thiourée de tout ou partie des groupements esters ou des groupements de protection du ou des radicaux amino ou du radical hydroxyle,

b) estérification ou salification par une base du ou des radicaux carboxyliques,

c) salification par un acide du ou des radicaux amino.

**2.** Procédé selon la revendication 1, caractérisé en ce que l'on utilise au départ un produit de formule (II) dans laquelle R' représente un radical alkyle linéaire ou ramifié, ayant de 1 à 4 atomes de carbone, éventuellement substitué par un ou plusieurs radicaux choisis parmi les radicaux carboxy libre ou estérifié, amino ou halogène.

**3.** Procédé selon la revendication 1 ou 2, caractérisé en ce que l'on utilise au départ un réactif de formule :

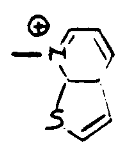

**4.** Procédé selon la revendication 1, 2 ou 3, caractérisé en ce que l'on prépare l'un quelconque des produits de formule (I) selon la revendication 1, dont les noms suivent :

- le 7-[(E)-3-[(6R,7R)-7-[[(2-amino 4-thiazolyl) [(Z)-(méthoxyimino)] acétyl] amino] 2-carboxy 8-oxo 5-thia 1-azabicyclo [4. 2. 0.] oct-2-èn-3-yl] 2-propényl] thiéno [2,3-b] pyridinium,
- le 7-[(E)-3-[(6R,7R)-7-[[(2-amino 4-thiazolyl) [(Z)-[(difluorométhoxy) imino]] acétyl] amino] 2-carboxy 8-oxo 5-thia 1-azabicyclo [4. 2. 0.] oct-2-èn-3-yl] 2-propényl] thiéno [2,3-b] pyridinium,

ainsi que leurs sels avec les métaux alcalin, alcalino-terreux, le magnésium, l'ammoniaque, les bases

organiques aminées, les acides, le cas échéant leurs sels internes et leurs esters facilement clivables.

5. Procédé de préparation de compositions pharmaceutiques, caractérisé en ce que l'on met, à titre de principe actif, l'un au moins des produits de formule (I), telle que définie à la revendication 1, ou l'un au moins de leurs sels avec les acides minéraux ou organiques pharmaceutiquement acceptables, sous une forme destinée à cet usage.

6. Procédé selon la revendication 5, caractérisé en ce que l'on utilise à titre de principe actif, l'un au moins des produits de formule (I), tels que définis à la revendication 2 ou 3.

7. Procédé selon la revendication 5, caractérisé en ce que l'on utilise à titre de principe actif, l'un au moins des produits de formule (I), tels que définis à la revendication 4.

**Claims**

**Claims for the following Contracting States : AT, BE, CH, DE, FR, GB, IT, LI, LU, NL, SE**

1. The products of general formula (I):

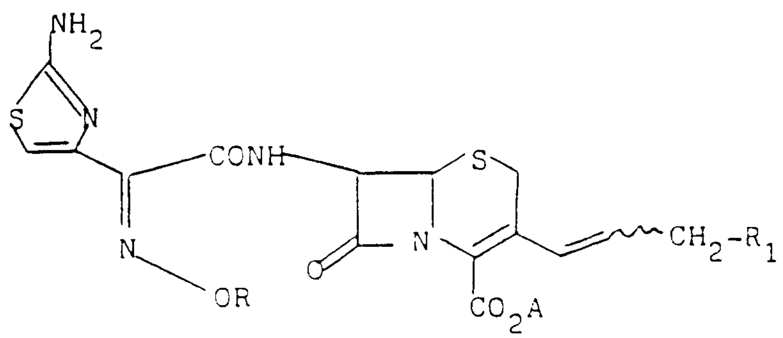

(I)

syn isomer

in which R represents a hydrogen atom or a linear or branched alkyl radical, an alkenyl, alkynyl or cycloalkyl radical having at most 6 carbon atoms, each of these radicals being optionally substituted by one or more of the following radicals:

optionally salified or esterified carboxy, methoxycarbonyl, ethoxycarbonyl, carbamoyl, dimethylcarbamoyl; amino; dimethylamino; methylamino; halogen; methoxy, ethoxy, propyloxy; methylthio, ethylthio; phenyl; tetrazolyl; phenylthio; tetrazolylthio, thiadiazolylthio optionally substituted by methyl; $R_1$ represents a radical chosen from the following radicals:

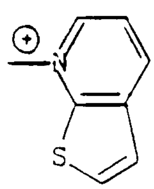 ; 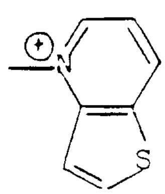 , 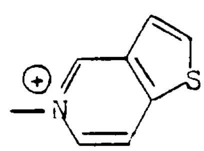 and 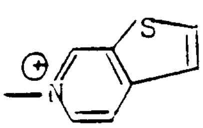

A represents a hydrogen atom, an equivalent of an alkali, alkaline-earth metal, magnesium, ammonium or an amino organic base or A represents the remainder of an easily cleavable ester group or $CO_2A$ represents $CO_2^{\ominus}$; the wavy line means that the $CH_2R_1$ group can be found in the E or Z position, as well as the salts of the products of formula (I) with mineral or organic acids.

2. The products of general formula (I) as defined in claim 1, in which R represents a linear or branched alkyl radical having 1 to 4 carbon atoms optionally substituted by one or more radicals chosen from free, esterified or salified carboxylic radicals, amino or halogen radicals.

**3.** The products of general formula (I) as defined in claim 1 or 2, in which $R_1$ represents a radical

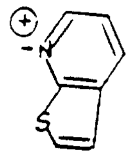

**4.** Any one of the products of formula (I) according to claim 1, of which the names follow:

- 7-[(E)-3-[(6R,7R)-7-[[(2-amino 4-thiazolyl) [(Z)-(methoxyimino)] acetyl] amino] 2-carboxy 8-oxo 5-thia 1-azabicyclo[4,2,O]oct-2-en-3-yl] 2-propenyl] thieno[2,3-b] pyridinium,
- 7-[(E)-3-[(6R,7R)-7[[(2-amino 4-thiazolyl) [(Z)-[(difluoromethoxy) imino]] acetyl] amino] 2-carboxy 8-oxo 5-thia 1-azabicyclo[4.2.0]oct-2-en-3-yl] 2-propenyl] thieno [2,3-b] pyridinium,

as well as their salts with alkali, alkaline-earth metals, magnesium, ammonium hydroxide, amino organic bases, acids, if appropriate their internal salts and their easily cleavable esters.

**5.** Preparation process for products of formula (I) as defined in claim 1, characterized in that a reagent chosen from the reagents of formulae:

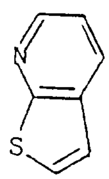 ; 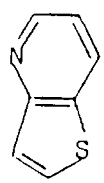 , 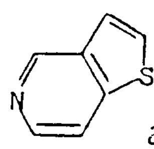 and 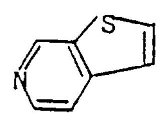

is reacted with a product of formula (II):

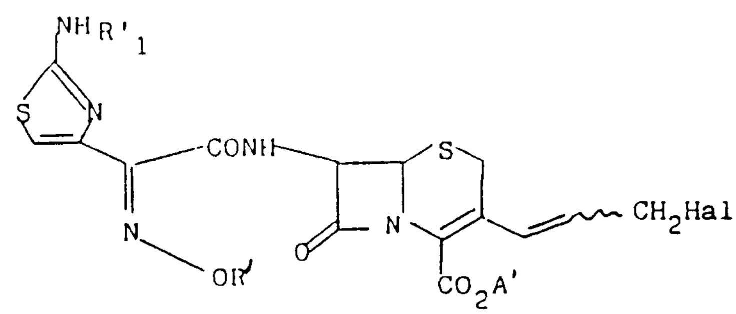

(II)

in which $R'_1$ represents a hydrogen atom or a protector group of the amino radical, R' represents either the values indicated in claim 1 for R, or a protector group of the hydroxyl radical, A' represents a hydrogen atom or the remainder of an easily eliminable ester group and Hal represents a halogen atom, in order to obtain a product of formula (III):

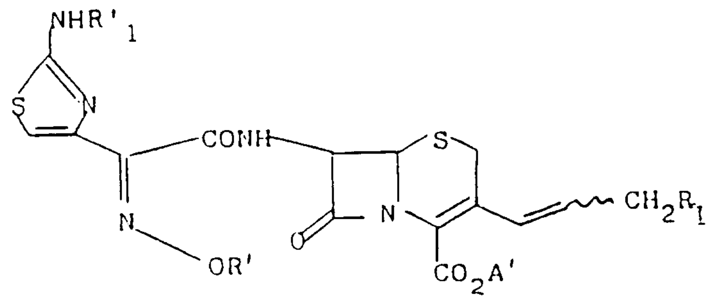

(III)

which if desired is separated into its E or Z isomers or the Z isomers are converted into E isomers and which products of formulae (III), if necessary or if desired, are subjected to one or more of the following reactions, in any order:

a) cutting by hydrolysis or by the action of thiourea of all or part of the ester groups or the protector groups of the amino radical or radicals or the hydroxyl radical,

b) esterification or salification by a base of the carboxylic radical or radicals,

c) salification by an acid of the amino radical or radicals.

**6.** As medicaments, the products corresponding to formula (I) as defined in claim 1, as well as their salts with pharmaceutically acceptable acids.

**7.** As medicaments, the products as defined in any one of claims 2 to 4, as well as their salts with pharmaceutically acceptable acids.

**8.** Pharmaceutical compositions containing, as active ingredient, at least one medicament according to one of claims 6 or 7.

**9.** As new industrial products, the products of formula (III) as defined in claim 5, in which $R'_1$ represents a protector group of the amino radical.

**Claims for the following Contracting State : ES**

**1.** Preparation process for the products of general formula (I):

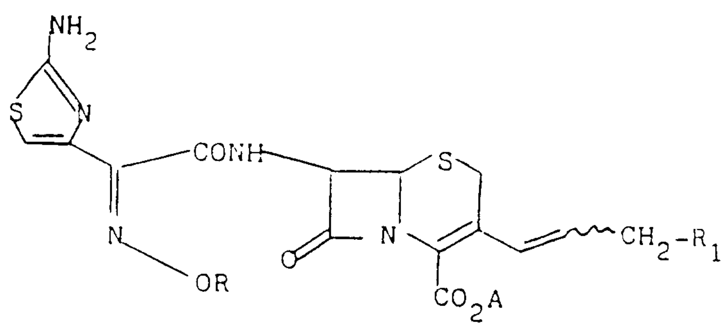

(I)

syn isomer

in which R represents a hydrogen atom or a linear or branched alkyl radical, an alkenyl, alkynyl or cycloalkyl radical having at most 6 carbon atoms, each of these radicals being optionally substituted by one or more of the following radicals:

optionally salified or esterified carboxy, methoxycarbonyl, ethoxycarbonyl, carbamoyl, dimethylcarbamoyl; amino; dimethylamino; methylamino; halogen; methoxy, ethoxy, propyloxy; methylthio, ethylthio; phenyl; tetrazolyl; phenylthio; tetrazolylthio, thiadiazolylthio optionally substituted by methyl; $R_1$ represents a radical chosen from the following radicals:

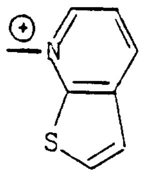 ; 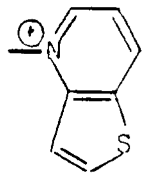 , 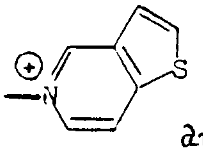 and 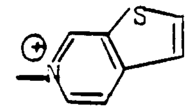

A represents a hydrogen atom, an equivalent of an alkali, alkaline-earth metal, magnesium, ammonium or an amino organic base or A represents the remainder of an easily cleavable ester group or $CO_2A$ represents $CO_2^{\ominus}$; the wavy line means that the $CH_2R_1$ group can be found in the E or Z position, as well as the salts of the products of formula (I) with mineral or organic acids, characterized in that a reagent chosen from the reagents of formulae:

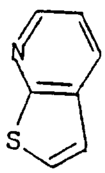 ; 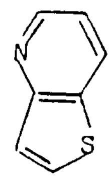 , 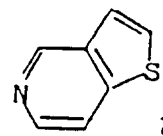 and 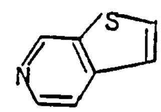

is reacted with a product of formula (II):

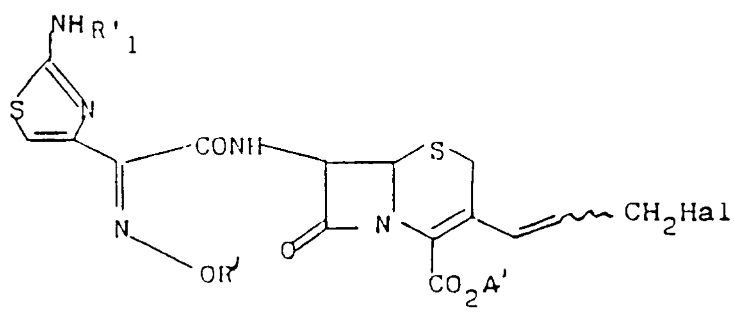

(II)

in which $R'_1$ represents a hydrogen atom or a protector group of the amino radical, R' represents either the values indicated previously, or a protector group of the hydroxyl radical, A' represents a hydrogen atom or the remainder of an easily eliminable ester group and Hal represents a halogen atom, in order to obtain a product of formula (III):

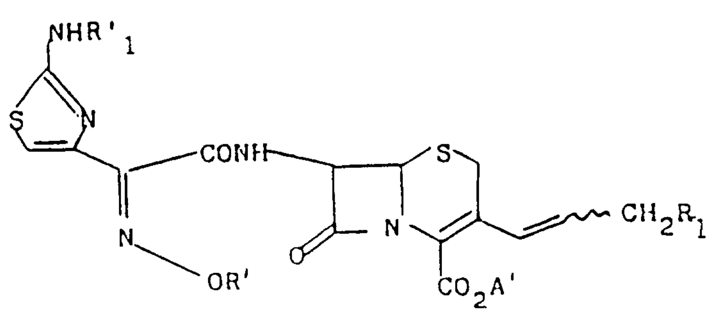

(III)

which if desired is separated into its E or Z isomers or the Z isomers are converted into E isomers and which products of formulae (III), if necessary or if desired, are subjected to one or more of the following reactions, in any order:

a) cutting by hydrolysis or by the action of thiourea of all or part of the ester groups or the protector groups of the amino radical or radicals or the hydroxyl radical,

b) esterification or salification by a base of the carboxylic radical or radicals,
c) salification by an acid of the amino radical or radicals.

**2.** Process according to claim 1, characterized in that a product of formula (II) is used at the start in which R' represents a linear or branched alkyl radical having 1 to 4 carbon atoms, optionally substituted by one or more radicals chosen from free or esterified carboxy radicals, amino or halogen radicals.

**3.** Process according to claim 1 or 2, characterized in that a reagent is used at the start of formula:

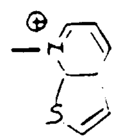

**4.** Process according to claim 1, 2 or 3, characterized in that any one of the products of formula (I) according to claim 1 is prepared, of which the names follow:
- 7-[(E)-3-[(6R,7R)-7-[[(2-amino 4-thiazolyl) [(Z)-(methoxyimino)] acetyl] amino] 2-carboxy 8-oxo 5-thia 1-azabicyclo[4,2,O]oct-2-en-3-yl] 2-propenyl] thieno[2,3-b] pyridinium,
- 7-[(E)-3-[(6R,7R)-7[[(2-amino 4-thiazolyl) [(Z)-[(difluoromethoxy) imino]] acetyl] amino] 2-carboxy 8-oxo 5-thia 1-azabicyclo[4.2.0]oct-2-en-3-yl] 2-propenyl] thieno [2,3-b] pyridinium,

as well as their salts with alkali, alkaline-earth metals, magnesium, ammonium hydroxide, amino organic bases, acids, if appropriate their internal salts and their easily cleavable esters.

**Claims for the following Contracting State : GR**

**1.** Preparation process for the products of general formula (I):

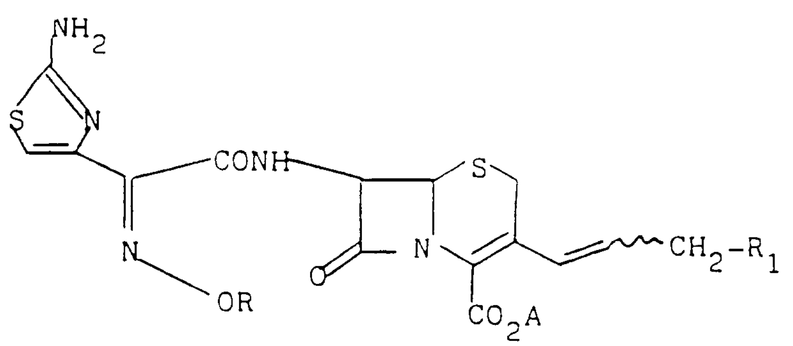

(I)

syn isomer
in which R represents a hydrogen atom or a linear or branched alkyl radical, an alkenyl, alkynyl or cycloalkyl radical having at most 6 carbon atoms, each of these radicals being optionally substituted by one or more of the following radicals:
optionally salified or esterified carboxy, methoxycarbonyl, ethoxycarbonyl, carbamoyl, dimethylcarbamoyl; amino; dimethylamino; methylamino; halogen; methoxy, ethoxy, propyloxy; methylthio, ethylthio; phenyl; tetrazolyl; phenylthio; tetrazolylthio, thiadiazolylthio optionally substituted by methyl; $R_1$ represents a radical chosen from the following radicals:

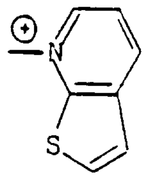 ; 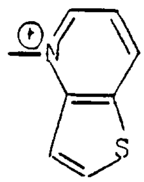 , 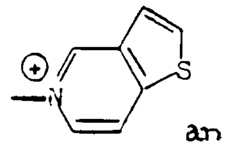 and 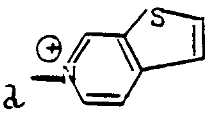

A represents a hydrogen atom, an equivalent of an alkali, alkaline-earth metal, magnesium, ammonium or an amino organic base or A represents the remainder of an easily cleavable ester group or $CO_2A$ represents $CO_2^{\ominus}$; the wavy line means that the $CH_2R_1$ group can be found in the E or Z position, as well as the salts of the products of formula (I) with mineral or organic acids, characterized in that a reagent chosen from the reagents of formulae:

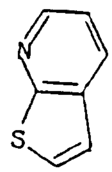 ; 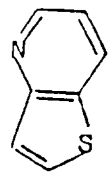 , 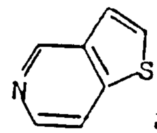 and 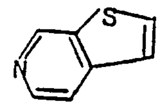

is reacted with a product of formula (II):

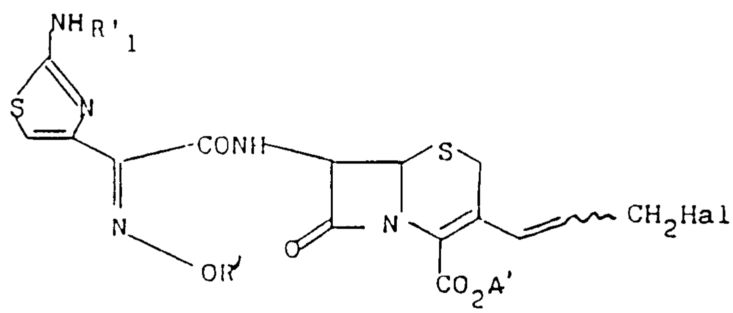

(II)

in which $R'_1$ represents a hydrogen atom or a protector group of the amino radical, R' represents either the values indicated previously, or a protector group of the hydroxyl radical, A' represents a hydrogen atom or the remainder of an easily eliminable ester group and Hal represents a halogen atom, in order to obtain a product of formula (III):

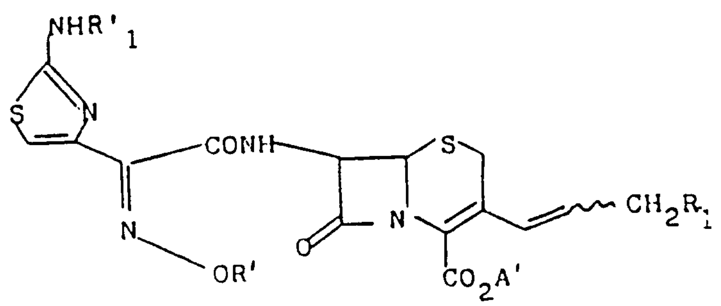

(III)

which if desired is separated into its E or Z isomers or the Z isomers are converted into E isomers and which products of formulae (III), if necessary or if desired, are subjected to one or more of the following reactions, in any order:

a) cutting by hydrolysis or by the action of thiourea of all or part of the ester groups or the protector groups of the amino radical or radicals or the hydroxyl radical,

b) esterification or salification by a base of the carboxylic radical or radicals,
c) salification by an acid of the amino radical or radicals.

2. Process according to claim 1, characterized in that a product of formula (II) is used at the start in which R' represents a linear or branched alkyl radical having 1 to 4 carbon atoms, optionally substituted by one or more radicals chosen from free or esterified carboxy radicals, amino or halogen radicals.

3. Process according to claim 1 or 2, characterized in that a reagent is used at the start of formula:

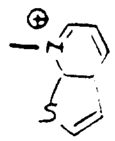

4. Process according to claim 1, 2 or 3, characterized in that any one of the products of formula (I) according to claim 1 is prepared, of which the names follow:
- 7-[(E)-3-[(6R,7R)-7-[[(2-amino 4-thiazolyl) [(Z)-(methoxyimino)] acetyl] amino] 2-carboxy 8-oxo 5-thia 1-azabicyclo[4,2,O]oct-2-en-3-yl] 2-propenyl] thieno[2,3-b] pyridinium,
- 7-[(E)-3-[(6R,7R)-7[[(2-amino 4-thiazolyl) [(Z)-[(difluoromethoxy) imino]] acetyl] amino] 2-carboxy 8-oxo 5-thia 1-azabicyclo[4.2.0]oct-2-en-3-yl] 2-propenyl] thieno [2,3-b] pyridinium,

as well as their salts with alkali, alkaline-earth metals, magnesium, ammonium hydroxide, amino organic bases, acids, if appropriate their internal salts and their easily cleavable esters.

5. Preparation process for pharmaceutical compositions, characterized in that at least one of the products of formula (I), as defined in claim 1, or at least one of their salts with pharmaceutically acceptable mineral or organic acids is used as active ingredient in a form intended for this use.

6. Process according to claim 5, characterized in that at least one of the products of formula (I), as defined in claim 2 or 3, is used as active ingredient.

7. Process according to claim 5, characterized in that at least one of the products of formula (I), as defined in claim 4, is used as active ingredient.

## Patentansprüche

## Patentansprüche für folgende Vertragsstaaten : AT, BE, CH, DE, FR, GB, IT, LI, LU, NL, SE

1. Verbindungen der allgemeinen Formel (I) sowie Salze der Verbindungen der Formel (I) mit anorganischen oder organischen Säuren:

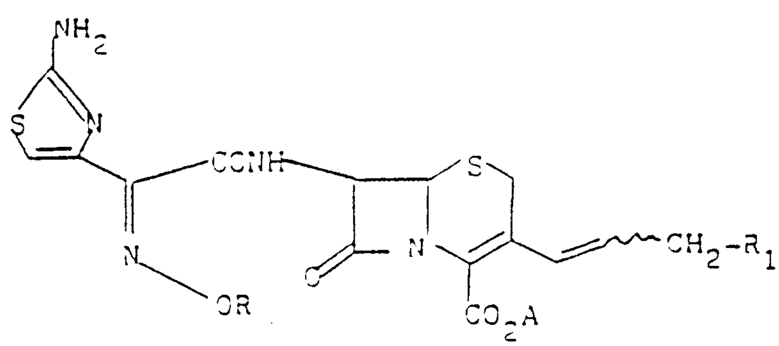

(I)

(syn-Isomer)

in der R ein Wasserstoffatom oder einen linearen oder verzweigten Alkylrest, einen Alkenylrest, einen Alkinylrest oder einen Cycloalkylrest mit höchstens 6 Kohlenstoffatomen bedeutet, wobei jeder dieser

Reste gegebenenfalls mit einem oder mehreren der nachstehenden Reste substituiert ist: gegebenenfalls als Salz vorliegender oder veresterter Carboxylrest, Methoxycarbonylrest, Ethoxycarbonylrest, Carbamoylrest, Dimethylcarbamoylrest; Aminorest; Dimethylaminorest; Methylaminorest; Halogenatom; Methoxyrest, Ethoxyrest, Propyloxyrest; Methylthiorest, Ethylthiorest; Phenylrest; Tetrazolylrest; Phenylthiorest; Tetrazolylthiorest, gegebenenfalls mit Methyl substituierter Thiadiazolylthiorest;
$R_1$ einen unter den nachstehenden Resten ausgewählten Rest bedeutet:

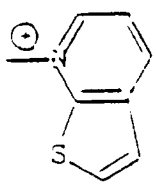 ; 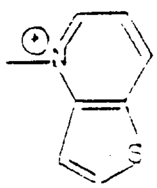 , 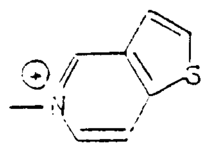 und 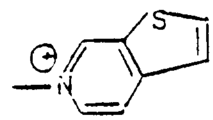

A ein Wasserstoffatom, ein Äquivalent eines Alkalimetalls, eines Erdalkalimetalls, von Magnesium, von Ammonium oder von einer organischen Aminbase bedeutet oder A den Rest einer leicht abspaltbaren Estergruppe bedeutet oder $CO_2A$ die Bedeutung $CO_2^-$ hat; und
die geschlängelte Linie anzeigt, daß sich die Gruppe $CH_2R_1$ in der E- oder Z-stellung befinden kann.

**2.** Verbindungen der allgemeinen Formel (I), wie sie in Anspruch 1 definiert ist, in der R einen linearen oder verzweigten Alkylrest mit 1 bis 4 Kohlenstoffatomen bedeutet, der gegebenenfalls mit einem oder mehreren, unter freien, veresterten oder in Salzform vorliegenden Carboxylresten, Aminoresten und Halogenatomen ausgewählten Resten substituiert sein kann.

**3.** Verbindungen der allgemeinen Formel (I), wie sie in Anspruch 1 oder 2 definiert ist, in der $R_1$ den nachstehenden Rest bedeutet:

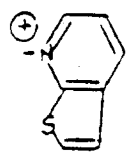

**4.** Verbindungen der Formel (I) nach Anspruch 1, nämlich:
7-[(E)-3-[(6R,7R)-7-[[(2-Amino-4-thiazolyl)-[(Z)-(methoxy)-imino]-acetyl]-amino]-2-carboxy-8-oxo-5-thia-1-azabicyclo[4.2.0]oct-2-en-3-yl]-2-propenyl]-thieno[2,3-b]-pyridinium und
7-[(E)-3-[(6R,7R)-7-[[(2-Amino-4-thiazolyl)-[(Z)-(difluormethoxy)-imino]-acetyl]-amino]-2-carboxy-8-oxo-5-thia-1-azabicyclo[4.2.0]oct-2-en-3-yl]-2-propenyl]-thieno[2,3-b]-pyridinium
sowie ihre Salze mit Alkalimetallen, Erdalkalimetallen, Magnesium, Ammoniak, organischen Aminbasen, Säuren, gegebenenfalls ihre inneren Salze und ihre leicht spaltbaren Ester.

**5.** Verfahren zur Herstellung der Verbindungen der Formel (I), wie sie in Anspruch 1 definiert ist, wobei das Verfahren dadurch gekennzeichnet ist, daß man ein unter den Reagentien mit den nachstehenden Formeln ausgewähltes Reagens:

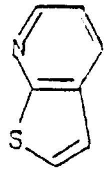 ; 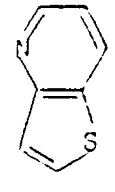 , 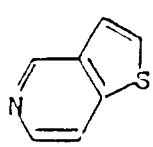 und 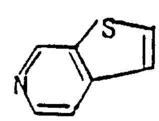

mit einer Verbindung der Formel (II) umsetzt:

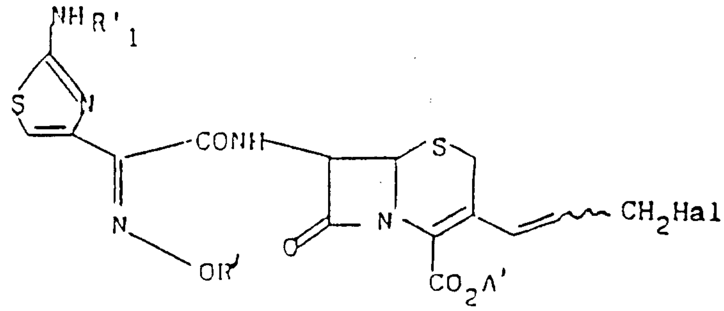

(II)

in der $R'_1$ ein Wasserstoffatom oder eine Schutzgruppe für einen Aminorest bedeutet;
R' entweder die in Anspruch 1 für R angegebene Bedeutung besitzt oder eine Schutzgruppe für einen Hydroxyrest bedeutet;
A' ein Wasserstoffatom oder den Rest einer leicht entfernbaren Estergruppe bedeutet; und
Hal ein Halogenatom bedeutet;
um eine Verbindung der Formel (III):

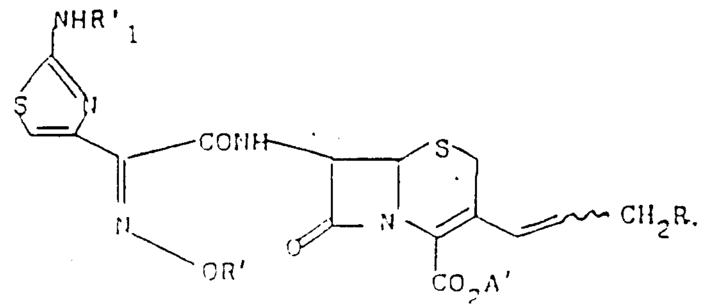

(III)

die man, falls gewünscht, in ihre E- und Z-Isomere trennen oder deren Z-Isomer man in das E-Isomer umwandeln kann, und eine Verbindung der Formel (III), die man, falls erforderlich oder falls gewünscht, einer oder mehreren der nachstehenden Reaktionen in einer beliebigen Reihenfolge unterwerfen kann, zu erhalten:

a) Abspaltung aller oder eines Teils der Estergruppen oder der Schutzgruppen des Aminorests oder der Aminoreste oder des Hydroxyrests durch Hydrolyse oder durch das Einwirken von Thioharnstoff;
b) Veresterung des Carboxylrests oder der Carboxylreste oder Bildung eines Salzes mit einer Base;
c) Bildung eines Salzes des Aminorests oder der Aminoreste mit einer Säure.

**6.** Verbindungen gemäß Formel (I), wie sie in Anspruch 1 definiert ist, sowie deren Salze mit pharmazeutisch verträglichen Säuren als Arzneistoffe.

**7.** Verbindungen, wie sie in einem der Ansprüche 2 bis 4 definiert sind, sowie deren Salze mit pharmazeutisch verträglichen Säuren als Arzneistoffe.

**8.** Pharmazeutische Zusammensetzungen, enthaltend als Wirkstoff mindestens einen Arzneistoff nach einem der Ansprüche 6 oder 7.

**9.** Verbindungen der Formel (III), wie sie in Anspruch 5 definiert ist, in der $R'_1$ eine Schutzgruppe des Aminorests bedeutet, als neue Industrieerzeugnisse.

**Patentansprüche für folgenden Vertragsstaat : ES**

**1.** Verfahren zur Herstellung von Verbindungen der allgemeinen Formel (I) sowie von Salzen der Verbindungen der Formel (I) mit anorganischen oder organischen Säuren:

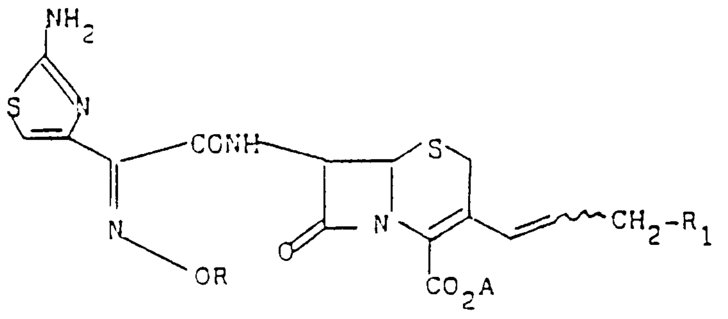

(I)

(syn-Isomer)

in der R ein Wasserstoffatom oder einen linearen oder verzweigten Alkylrest, einen Alkenylrest, einen Alkinylrest oder einen Cycloalkylrest mit höchstens 6 Kohlenstoffatomen bedeutet, wobei jeder dieser Reste gegebenenfalls mit einem oder mehreren der nachstehenden Reste substituiert ist:

gegebenenfalls als Salz vorliegender oder veresterter Carboxylrest, Methoxycarbonylrest, Ethoxycarbonylrest, Carbamoylrest, Dimethylcarbamoylrest; Aminorest; Dimethylaminorest; Methylaminorest; Halogenatom; Methoxyrest, Ethoxyrest, Propyloxyrest; Methylthiorest, Ethylthiorest; Phenylrest; Tetrazolylrest; Phenylthiorest; Tetrazolylthiorest, gegebenenfalls mit Methyl substituierter Thiadiazolylthiorest;

$R_1$ einen unter den nachstehenden Resten ausgewählten Rest bedeutet:

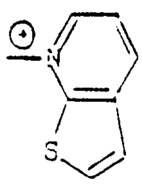 ; 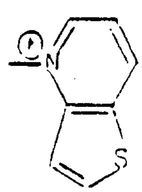 , 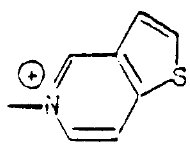 und 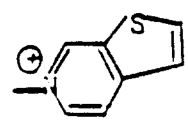

A ein Wasserstoffatom, ein Äquivalent eines Alkalimetalls, eines Erdalkalimetalls, von Magnesium, von Ammonium oder von einer organischen Aminbase bedeutet oder A den Rest einer leicht abspaltbaren Estergruppe bedeutet oder $CO_2A$ die Bedeutung $CO_2^-$ hat; und

die geschlängelte Linie anzeigt, daß sich die Gruppe $CH_2R_1$ in der E- oder Z-Stellung befinden kann,

wobei das Verfahren dadurch gekennzeichnet ist, daß man ein unter den Reagentien mit den nachstehenden Formeln ausgewähltes Reagens:

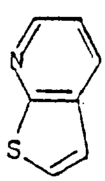 ; 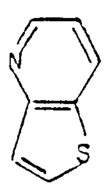 , 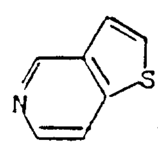 und 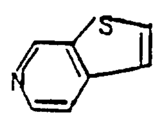

mit einer Verbindung der Formel (II) umsetzt:

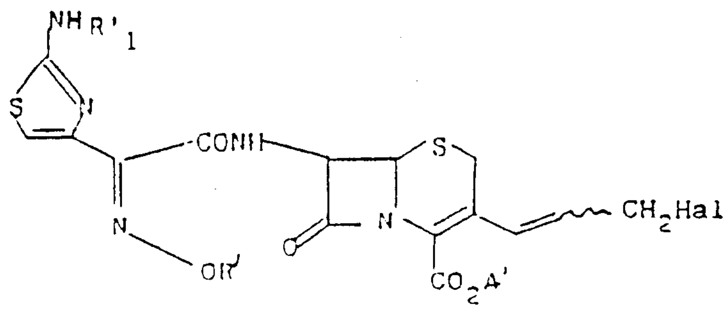

(II)

in der $R'_1$ ein Wasserstoffatom oder eine Schutzgruppe für einen Aminorest bedeutet;
R' entweder die vorstehend angegebene Bedeutung besitzt oder eine Schutzgruppe für einen Hydroxyrest bedeutet;
A' ein Wasserstoffatom oder den Rest einer leicht entfernbaren Estergruppe bedeutet; und
Hal ein Halogenatom bedeutet;
um eine Verbindung der Formel (III):

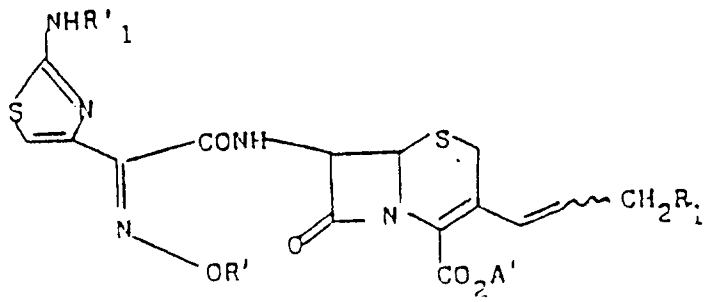

(III)

die man, falls gewünscht, in ihre E- und Z-Isomere trennen oder deren Z-Isomer man in das E-Isomer umwandeln kann, und ein Produkt der Formel (III), das man, falls erforderlich oder falls gewünscht, einer oder mehreren der nachstehenden Reaktionen in einer beliebigen Reihenfolge unterwerfen kann, zu erhalten:

a) Abspaltung aller oder eines Teils der Estergruppen oder der Schutzgruppen des Aminorests oder der Aminoreste oder des Hydroxyrests durch Hydrolyse oder durch das Einwirken von Thioharnstoff;
b) Veresterung des Carboxylrests oder der Carboxylreste oder Bildung eines Salzes mit einer Base;
c) Bildung eines Salzes des Aminorests oder der Aminoreste mit einer Säure.

**2.** Verfahren nach Anspruch 1, dadurch gekennzeichnet, daß man als Ausgangsmaterial eine Verbindung der Formel (II) einsetzt, in der R' einen linearen oder verzweigten Alkylrest mit 1 bis 4 Kohlenstoffatomen bedeutet, der gegebenenfalls mit einem oder mehreren, unter freien oder veresterten Carboxylresten, Aminoresten und Halogenatomen ausgewählten Resten substituiert sein kann.

**3.** Verfahren nach einem der Ansprüche 1 oder 2, dadurch gekennzeichnet, daß man als Ausgangsmaterial ein Reagens der nachstehenden Formel einsetzt:

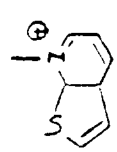

**4.** Verfahren nach Anspruch 1, 2 oder 3, dadurch gekennzeichnet, daß man eine der Verbindungen der Formel (I) nach Anspruch 1, nämlich:

7-[(E)-3-[(6R,7R)-7-[[(2-Amino-4-thiazolyl)-[(Z)-(methoxy)-imino]-acetyl]-amino]-2-carboxy-8-oxo-5-thia-1-azabicyclo[4.2.0]oct-2-en-3-yl]-2-propenyl]-thieno[2,3-b]-pyridinium und
7-[(E)-3-[(6R,7R)-7-[[(2-Amino-4-thiazolyl)-[(Z)-(difluormethoxy)-imino]-acetyl]-amino]-2-carboxy-8-oxo-5-thia-1-azabicyclo[4.2.0]oct-2-en-3-yl]-2-propenyl]-thieno[2,3-b]-pyridinium
sowie ihre Salze mit Alkalimetallen, Erdalkalimetallen, Magnesium, Ammoniak, organischen Aminbasen, Säuren, gegebenenfalls ihre inneren Salze und ihre leicht spaltbaren Ester herstellt.

**Patentansprüche für folgenden Vertragsstaat : GR**

1. Verfahren zur Herstellung von Verbindungen der allgemeinen Formel (I) sowie von Salzen der Verbindungen der Formel (I) mit anorganischen oder organischen Säuren:

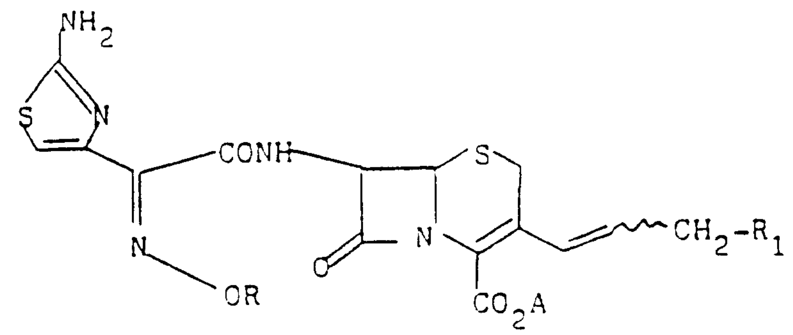

(I)

(syn-Isomer)
in der R ein Wasserstoffatom oder einen linearen oder verzweigten Alkylrest, einen Alkenylrest, einen Alkinylrest oder einen Cycloalkylrest mit höchstens 6 Kohlenstoffatomen bedeutet, wobei jeder dieser Reste gegebenenfalls mit einem oder mehreren der nachstehenden Reste substituiert ist:
gegebenenfalls als Salz vorliegender oder veresterter Carboxylrest, Methoxycarbonylrest, Ethoxycarbonylrest, Carbamoylrest, Dimethylcarbamoylrest; Aminorest; Dimethylaminorest; Methylaminorest; Halogenatom; Methoxyrest, Ethoxyrest, Propyloxyrest; Methylthiorest, Ethylthiorest; Phenylrest; Tetrazolylrest; Phenylthiorest; Tetrazolylthiorest, gegebenenfalls mit Methyl substituierter Thiadiazolylthiorest;
$R_1$ einen unter den nachstehenden Resten ausgewählten Rest bedeutet:

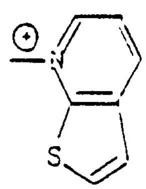 ; 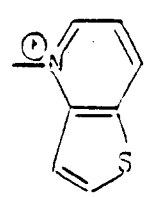 , 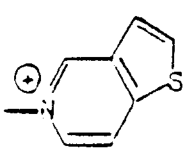 und 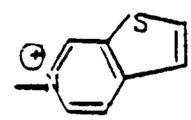

A ein Wasserstoffatom, ein Äquivalent eines Alkalimetalls, eines Erdalkalimetalls, von Magnesium, von Ammonium oder von einer organischen Aminbase bedeutet oder A den Rest einer leicht abspaltbaren Estergruppe bedeutet oder $CO_2A$ die Bedeutung $CO_2^-$ hat; und
die geschlängelte Linie anzeigt, daß sich die Gruppe $CH_2R_1$ in der E- oder Z-stellung befinden kann, wobei das Verfahren dadurch gekennzeichnet ist, daß man ein unter den Reagentien mit den nachstehenden Formeln ausgewähltes Reagens:

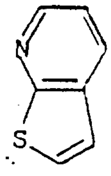 ; 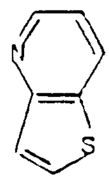 , 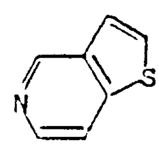 und 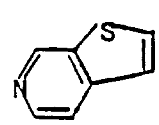

mit einer Verbindung der Formel (II) umsetzt:

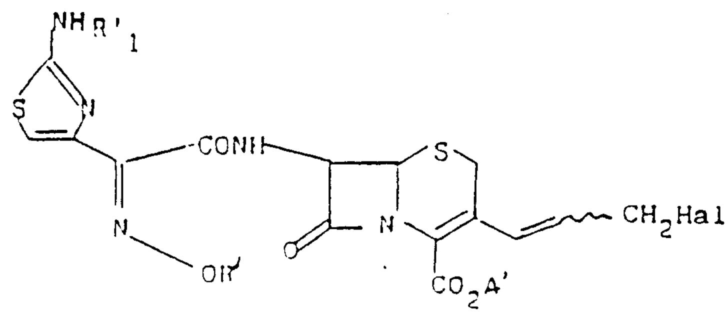

(II)

in der $R'_1$ ein Wasserstoffatom oder eine Schutzgruppe für einen Aminorest bedeutet;
R' entweder die vorstehend angegebene Bedeutung besitzt oder eine Schutzgruppe für einen Hydroxyrest bedeutet; A' ein Wasserstoffatom oder den Rest einer leicht entfernbaren Estergruppe bedeutet; und
Hal ein Halogenatom bedeutet;
um eine Verbindung der Formel (III):

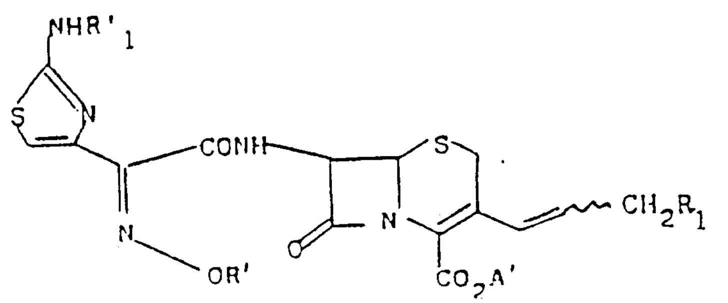

(III)

die man, falls gewünscht, in ihre E- und Z-Isomere trennen oder deren Z-Isomer man in das E-Isomer umwandeln kann, und ein Produkt der Formel (III), das man, falls erforderlich oder falls gewünscht, einer oder mehreren der nachstehenden Reaktionen in einer beliebigen Reihenfolge unterwerfen kann, zu erhalten:

a) Abspaltung aller oder eines Teils der Estergruppen oder der Schutzgruppen des Aminorests oder der Aminoreste oder des Hydroxyrests durch Hydrolyse oder durch das Einwirken von Thioharnstoff;
b) Veresterung des Carboxylrests oder der Carboxylreste oder Bildung eines Salzes mit einer Base;
c) Bildung eines Salzes des Aminorests oder der Aminoreste mit einer Säure.

**2.** Verfahren nach Anspruch 1, dadurch gekennzeichnet, daß man als Ausgangsmaterial eine Verbindung der Formel (II) einsetzt, in der R' einen linearen oder verzweigten Alkylrest mit 1 bis 4 Kohlenstoffatomen bedeutet, der gegebenenfalls mit einem oder mehreren, unter freien oder veresterten Carboxylresten, Aminoresten und Halogenatomen ausgewählten Resten substituiert sein kann.

**3.** Verfahren nach einem der Ansprüche 1 oder 2, dadurch gekennzeichnet, daß man als Ausgangsmaterial ein Reagens der nachstehenden Formel einsetzt:

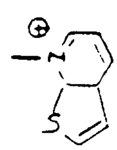

4. Verfahren nach Anspruch 1, 2 oder 3, dadurch gekennzeichnet, daß man eine der Verbindungen der Formel (I) nach Anspruch 1, nämlich:
7-[(E)-3-[(6R,7R)-7-[[(2-Amino-4-thiazolyl)-[(Z)-(methoxy)-imino]-acetyl]-amino]-2-carboxy-8-oxo-5-thia-1-azabicyclo[4.2.0]oct-2-en-3-yl]-2-propenyl]-thieno[2,3-b]-pyridinium und
7-[(E)-3-[(6R,7R)-7-[[(2-Amino-4-thiazolyl)-[(Z)-(difluormethoxy)-imino]-acetyl]-amino]-2-carboxy-8-oxo-5-thia-1-azabicyclo[4.2.0]oct-2-en-3-yl]-2-propenyl]-thieno[2,3-b]-pyridinium
sowie ihre Salze mit Alkalimetallen, Erdalkalimetallen, Magnesium, Ammoniak, organischen Aminbasen, Säuren, gegebenenfalls ihre inneren Salze und ihre leicht spaltbaren Ester herstellt.

5. Verfahren zur Herstellung pharmazeutischer Zusammensetzungen, dadurch gekennzeichnet, daß man als Wirkstoff mindestens eine Verbindung der Formel (I), wie sie in Anspruch 1 definiert ist, oder mindestens eines ihrer Salze mit pharmazeutisch verträglichen anorganischen oder organischen Säuren in eine für diese Verwendung geeignete Form bringt.

6. Verfahren nach Anspruch 5, dadurch gekennzeichnet, daß man als Wirkstoff mindestens eine der Verbindungen der Formel (I), wie sie in Anspruch 2 oder 3 definiert ist, verwendet.

7. Verfahren nach Anspruch 5, dadurch gekennzeichnet, daß man als Wirkstoff mindestens eine der Verbindungen der Formel (I), wie sie in Anspruch 4 definiert ist, verwendet.